# EUROPEAN PATENT APPLICATION

(11) **EP 2 740 490 A1**
(43) Date of publication of application: **11.06.2014**
(21) Application number: 14157123.2
(22) Date of filing: 03.10.2008
(51) Int. Cl.: A61K 39/395, A61K 45/06, C07K 16/30

(54) **Treatment of proliferative disorders using antibodies to PSMA**

(30) Priority: 03.10.2007 US 977364 P
(62) Divisional of application: 08836466.6
(71) Applicant: Cornell University, Ithaca, NY 14850 (US)
(72) Inventor: Bander, Neil H., Chappaqua, NY New York 10514 (US)
(74) Representative: Fleck, Barbara

(57) **Abstract**

Methods of treating cancer in a patient are provided. In some embodiments the method comprises administering an antibody that is capable of binding to the extracellular domain of PSMA. In some embodiments, the method comprises restricting folate intake by the patient. Methods of monitoring cancer therapy are provided as well as kits for treating cancer and kits for monitoring cancer therapy.

## Description

### FIELD OF THE INVENTION

The present invention relates to treatment of proliferative disorders wherein one or more cell types associated with the disorder express prostate specific membrane antigen (PSMA).

### BACKGROUND

Prostate Specific Membrane Antigen (PSMA) expression is highly associated with prostate cancer and with other solid tumors; however any functional role of PSMA in cancer has been elusive.

PSMA is present on the cell surface of some normal prostatic epithelial cells, normal renal proximal tubular cells, proximal small bowel and some astrocytes (found in the brain). PSMA is highly upregulated/overexpressed on prostate cancer (Pca) cells. Expression levels of PSMA increase along with prostate cancer progression and PSMA levels in early stage prostate cancer predict a higher likelihood of recurrence. Furthermore, virtually all solid tumors express PSMA in their tumor neo-vasculature whereas normal vascular endothelium is PSMA-negative. Beyond the correlation of PSMA expression with prostate cancer and in non-prostate cancer neo-vasculature, no functional role for PSMA in cancer biology has been demonstrated. In addition, it has been reported that PSMA may, somewhat counter-intuitively, diminish cell motility and invasion.

PSMA is identical to folate hydrolase 1 (found in intestine) and NAALADase (found in brain) and possesses glutamate carboxypeptidase enzymatic activity. PSMA can hydrolyze a dipeptide, such as aspartic acid-glutamate into its constituent individual amino acids, a process thought to be involved in the process of neurotransmission and possibly various neurodegenerative disorders. As a result, researchers are developing small molecule inhibitors as possible neuro-therapeutics. PSMA also has folate hydrolase activity which allows it to cleave glutamate residues from folylpolyglutamate resulting in folylmonoglutamate.

Folylpolyglutamate is the natural form of folate found in food and is unable to cross the cell membrane or the intestinal epithelium, whereas folylmonoglutamate can be transported across cell membranes and the intestine. It has been recently shown that small molecule PSMA enzyme inhibitors could slow the growth rate of PSMA-expressing Pca cells *in vitro.* (Yao and Bacich, the Prostate 66:867 (2006)). However, use of PSMA enzyme inhibitors in the past has failed to have any meaningful effect on tumor cell growth in animal models. Previous attempts of enzymatic blockade in the absence of other cytotoxic agents had no anti-tumor effect in animal models. Nanus, D.M., Milowsky, M.I., Kostakoglu, L., Vallabahajosula, S., and Goldsmith, S.J.: Targeted systemic therapy of prostate cancer with a monoclonal antibody to prostate specific membrane antigen (PSMA). Seminary in Oncology, 2003; 30: 667-676). Whole body folate metabolism is critical for normal physiological processes. However, small molecule inhibitors of PSMA/folate hydrolase have a much greater volume of distribution that includes both the extracellular and intracellular space as well as rapid passage through the renal tubules and have inhibitory impact on both tumor sites *and* normal tissues, thereby disrupting normal body folate metabolism.

Prostate cancer is one of the most common causes of cancer deaths in American males. In 2007, approximately 219,000 new cases are expected to be diagnosed as well as 27,000 deaths due to this disease (NCI SEER data; Cancer Facts and Figures, American Cancer Society). There is currently very limited treatment for prostate cancer once it has metastasized (spread beyond the prostate). Systemic therapy is limited to various forms of androgen (male hormone) deprivation. While most patients will demonstrate initial clinical improvement, virtually inevitably, androgen-independent cells develop. Endocrine therapy is thus palliative, not curative. (Eisenberger M. A., et al. (1998) NEJM 339:1036-42). Median overall survival in these patients where androgen-independent cells have developed was 28-52 months from the onset of hormonal treatment (Eisenberger M. A., et al. (1998) supra.). Subsequent to developing androgen-independence, only taxane-based (i.e., docetaxel) chemotherapy has been shown to provide a survival benefit, with a median survival of 19 months. Once patients fail to respond to docetaxel, median survival is 12 months.

Where prostate cancer is localized and the patient's life expectancy is 10 years or more, radical prostatectomy offers the best chance for eradication of the disease. Historically, the drawback of this procedure is that many cancers had spread beyond the bounds of the operation by the time they were detected. However, the use of prostate-specific antigen testing has permitted early detection of prostate cancer. As a result, surgery is less extensive with fewer complications. Patients with bulky, high-grade tumors are less likely to be successfully treated by radical prostatectomy. Radiation therapy has also been widely used as an alternative to radical prostatectomy. Patients generally treated by radiation therapy are those who are older and less healthy and those with higher-grade, more clinically advanced tumors. However, after surgery or radiation therapy, if there are detectable serum prostate-specific antigen concentrations, persistent cancer is indicated. In many cases, prostate-specific antigen concentrations can be reduced by radiation treatment. However, this concentration often increases again within two years.

For treatment of patients with locally advanced disease, hormonal therapy before or following radical prostatectomy or radiation therapy has been utilized. Orchiectomy reduces serum testosterone concentrations, while estrogen treatment is similarly beneficial.

Monoclonal antibodies which recognize PSMA have been developed, including 7E11, which binds to the intracellular domain. (Horoszewicz et al. (1987) Anticancer Res. 7:927-936, U.S. Pat. Nos. 5,162,504; 6,107,090; US 6,150,508; and 7,045,605), and other anti-PSMA antibodies that bind the extracellular domain.

### SUMMARY OF THE INVENTION

Provided herein are methods of treating cancer in a patient. In some embodiments, the method comprises administering an antibody or antigen binding fragment thereof to the patient (e.g., a patient having been diagnosed with cancer), wherein the antibody or antigen binding fragment thereof is capable of binding to the extracellular domain of PSMA and inhibiting enzymatic activity of the PSMA, and restricting intake of folate by the patient. In some embodiments folate intake by the patient is restricted by proscribing folate containing dietary supplements or intake of folate by the patient is restricted to 400 µg per day or less, or such that the serum level of folate in the patient is 10 nmol/L or less, or such that the red blood cell (RBC) folate level in the patient is 300 nmol/L or less or combinations thereof. In some embodiments, the antibody or antigen binding fragment thereof is unlabeled.

In other embodiments, the method of treating cancer in a patient comprises administering an antibody or antigen binding fragment thereof to a patient, wherein the antibody or antigen binding fragment thereof is capable of binding to an extracellular domain of PSMA and inhibiting enzymatic activity of the PSMA and measuring the blood level of folate in the patient.

In some embodiments, the method of treating cancer in a patient comprises administering a first antibody or antigen binding fragment thereof to the patient wherein the first antibody or antigen binding fragment thereof is capable of binding to the extracellular domain of PSMA and inhibiting enzymatic activity of the PSMA, restricting intake of folate by the patient, and administering a second antibody or antigen binding fragment thereof to the patient that is capable of binding to an extracellular domain (or another extracellular epitope) of PSMA. In some embodiments, folate intake is restricted such that folate intake is 400 µg per day or less, or such that the serum level of folate in the patient is 10 nmol/L or less, or such that the red blood cell (RBC) folate level in the patient is 300 nmol/L or less. In some embodiments, the second antibody or antigen binding fragment thereof is capable of binding to a different epitope of the extracellular domain of PSMA, wherein the second antibody or antigen binding fragment thereof is conjugated to a cytotoxic or radioisotopic agent or is capable of eliciting a secondary immune response. In some embodiments, the first dose of the second antibody is administered two to four weeks after intake of folate has been restricted.

In some embodiments, the method of treating cancer in a patient comprises restricting intake of folate by the patient and administering an antibody or antigen binding fragment thereof to the patient, wherein the antibody or antigen binding fragment thereof is capable of binding to an extracellular domain of PSMA, and wherein the first dose of the antibody or antigen binding fragment thereof is administered one to five days after cell surface levels of PSMA on PSMA expressing cells of the patient has increased by five fold or more. In some embodiments, folate intake is restricted such that folate intake is 400 µg per day or less, or such that the serum level of folate in the patient is 10 nmol/L or less, or such that the red blood cell (RBC) folate level in the patient is 300 nmol/L or less.

In some embodiments, the method of treating cancer in a patient comprises measuring surface levels of PSMA on PSMA expressing cells of the patient. In some embodiments, the first dose of the antibody or antigen binding fragment thereof is administered when cell surface levels of PSMA on PSMA expressing cells of the patient has increased by five fold or more.

Methods of treating prostate cancer in a patient are also provided. In some embodiments, the method comprises administering hormonal therapy to the patient, wherein the patient has been diagnosed with micro-metastatic prostate cancer (sometimes referred to as stage D0 or prostate specific antigen-only relapse or biochemical relapse) and administering an antibody or antigen binding fragment thereof that is capable of binding to an extracellular domain of PSMA wherein the antibody or antigen binding fragment thereof is conjugated to Lutetium-177 or other short-range alpha or beta-radioisotope, and wherein a first dose of the antibody or antigen binding fragment thereof is administered one day to three weeks after hormonal therapy is begun.

In some embodiments of methods of treating prostate cancer, the method comprises administering hormonal therapy a patient (e.g., a patient having been diagnosed with prostate cancer) and administering an antibody or antigen binding fragment thereof to the patient, wherein the antibody or antigen binding fragment thereof is capable of binding to an extracellular domain of PSMA, and wherein a first dose of the antibody or antigen binding fragment thereof is administered to the patient one day to four weeks after serum testosterone levels of the patient have reached 50 ng/mL or less.

Provided herein are methods of monitoring cancer therapy in a patient. In some embodiments, the method comprises measuring blood levels of folate in the patient, wherein folate intake by the patient is being restricted such that folate intake is 400 µg per day or less, or such that the serum level of folate in the patient is 10 nmol/L or less, or such that the red blood cell (RBC) folate level in the patient is 300 nmol/L or less, and wherein the patient has received at least one dose of an antibody or antigen binding fragment thereof that is capable of binding to an extracellular domain of PSMA and inhibiting enzymatic activity of the PSMA.

In some embodiments of monitoring cancer therapy in a patient, PSMA activity of PSMA expressing cells of a patient is measured, wherein folate intake by the patient is being restricted such that folate intake is 400 µg per day or less, or such that the serum level of folate in the patient is 10 nmol/L or less, or such that the red blood cell (RBC) folate level in the patient is 300 nmol/L or less, and wherein the patient has received at least one dose of an antibody or antigen binding fragment thereof that is capable of binding to an extracellular domain of PSMA and inhibiting enzymatic activity of the PSMA.

Also provided herein are kits for treating cancer. In some embodiments, the kit comprises an antibody or antigen binding fragment thereof that is capable of binding to an extracellular domain of PSMA and inhibiting enzymatic activity of the PSMA and instructions for restricting intake of folate by the patent and/or for monitoring blood levels of folate in the patient.

Also provided herein are kits for monitoring cancer therapy in a patient. In some embodiments, the kit comprises a tissue or blood collection apparatus, a PSMA activity detection reagent, and instructions for testing tissue or blood obtained from the patient using the PSMA activity detection reagent.

In some embodiments, the kit comprises a blood collection apparatus, a folate detection reagent, and instructions for testing red blood cells or serum obtained from the patient using the folate detection reagent. In some embodiments, the kit further comprises instructions for reducing folate intake by the patient.

In some embodiments of the methods and kits provided herein, the antibody or antigen binding fragment thereof that is capable of binding to the extracellular domain of PSMA and/or antibody or antigen binding fragment thereof that is capable of binding to the extracellular domain of PSMA and inhibiting enzymatic activity of the PSMA is unlabeled or naked antibody or antigen binding fragment thereof. In other embodiments, the antibody or antigen binding fragment thereof is labeled as described below.

Also provided herein are kits for treating early stage prostate cancer in a patient. In some embodiments, the kits comprise (i) an antibody or antigen binding fragment thereof that is capable of binding to an extracellular domain of PSMA wherein the antibody or antigen binding fragment thereof is conjugated to Lutetium-177; and (ii) instructions for administering a first dose of the antibody or antigen binding fragment thereof either one day to four weeks after a hormonal therapy has begun or one day to four weeks after serum testosterone levels of the patient have reached 50 ng/mL or less.

Also provided herein are kits for treating cancer in a patient. In some embodiments, the kits comprise (i) an unlabeled antibody or antigen binding fragment thereof that is capable of binding to an extracellular domain of PSMA and inhibiting enzymatic activity of the PSMA; and (ii) instructions for administering the unlabeled antibody or antigen binding fragment thereof in conjunction with restricting intake of folate by the patient.

The various embodiments described herein can be complimentary and can be combined or used together in a manner understood by the skilled person in view of the teachings contained herein.

### BRIEF DESCRIPTION OF THE DRAWINGS

FIG. 1 shows a histogram of PSMA expression levels on LNCaP cells grown in standard RPMI media and labeled with J591 antibody (1 = negative control, no J591 antibody,; 2 = cells grown in standard RPMI media containing 10% fetal calf serum (FCS), 3, 4, and 5 = cells grown in charcoal stripped media for 1, 2, and 3 weeks, respectively).
FIG. 2 shows a histogram of PSMA expression levels on MDA-PCa-2b cells grown in standard RPMI media and labeled with J591 antibody (1 = negative control, no J591 antibody; 2 = cells grown in standard RPMI media containing 10% fetal calf serum (FCS), 3 and 4 = cells grown in charcoal stripped media for 2 and 3 weeks, respectively).
FIG. 3 shows an increasing growth rate of LNCaP cells as the concentration of folic acid increases.
FIG. 4 shows the relative PSMA enzymatic activity of LNCaP cells in the presence of the indicated concentration of anti-PSMA antibodies J415, J591, and 7E11.
FIG. 5 shows the level of PSMA expression increases in the human kidney carcinoma cell line SK-RC-31 as the concentrations of folic acid in the media decrease towards physiological (10 nmol/L or 4.4 ng/mL).
FIG. 6 shows the level of PSMA expression in the human kidney carcinoma cell line SK-RC-42 in the presence of the indicated concentrations of folate.
FIG. 7 shows the level of PSMA expression increases in the human kidney carcinoma cell line SK-RC-39 as the concentrations of folic acid in the media decrease towards physiological (10 nmol/L).
FIG. 8 shows the level of PSMA expression increases in the human kidney carcinoma cell line SK-RC-06 as the concentrations of folic acid in the media decrease towards physiological (10 nmol/L).
FIG. 9 shows the level of PSMA expression increases in the human prostate cancer cell line Cwr22rv1 as the concentrations of folic acid in the media decrease towards physiological (10 nmol/L).
FIG. 10 shows the level of PSMA expression increases in the human prostate cancer cell line PC3 as the-concentrations of folic acid in the media decrease towards physiological (10 nmol/L).
FIG. 11 shows the level of PSMA expression in the human prostate cancer cell line LNCaP in the presence of the indicated concentrations of folate.
FIG. 12 shows a graph of cell number versus concentration of folate in cell culture media in the presence of the indicated concentrations of taxotere.
FIG. 13 shows a graph of tumor size versus time in mice treated with 3 different anti-PSMA antibodies, J591, 7E11 and J415, where mice in groups A and B have also been provided with folylpolyglutamate supplemented water.

### DETAILED DESCRIPTION OF THE INVENTION

Provided herein are methods of treating cancer and methods of monitoring the treatment of cancer. In addition, kits for treating cancer as well as kits for monitoring the treatment of cancer in a patient are provided. As described herein, in some embodiments, treatment comprises administering antibodies or antigen binding fragments thereof that are capable of binding to the extracellular domain of PSMA and inhibiting enzymatic activity of the PSMA As demonstrated herein, administration of such antibodies or antigen binding fragments thereof inhibit growth of PSMA-expressing tumors *in vivo* when intake of folate by the patient is restricted. Surprisingly, the antibody or antigen binding fragment thereof can be unlabeled, or naked, antibody or antigen binding fragment thereof.

Antigen or antigen binding fragments thereof, by virtue of their physical size, composition and charge, would have a biodistribution limited to the extracellular space, unable to cross the blood brain barrier and not subject to glomerular filtration or renal tubule excretion thereby preventing contact with PSMA/folate hydrolase in sites of important normal function such as the proximal tubule of the kidney, the proximal small bowel and the brain. Antibody or antigen binding fragments thereof are expected to have access to bind PSMA/folate hydrolase where present on tumor or tumor-related tissue as these are the only sites where PSMA/folate hydrolase is exposed to antibody or antigen binding fragments thereof in the circulation. This imparts a functional specificity to the action of antibody or antigen binding fragments thereof whereby folate metabolism is affected in tumor sites but not in normal tissues. As a result, whole body folate metabolism which is critical for normal physiological processes is unaffected whereas tumor folate metabolism can be specifically inhibited.

However, as described herein, lower doses of small molecule PSMA/folate hydrolase inhibitors can be added to or combined with antibody or antigen binding fragments thereof that inhibit the enzymatic activity of PSMA/folate hydrolase to achieve additive or synergistic inhibition at tumor sites while leading to only minimal inhibition in normal tissues so as not to disrupt normal folate metabolism.

In addition, as demonstrated herein, reduction in the amount of folate results in an increase in the amount of PSMA on the surface of PSMA expressing cells. Methods of treating cancer are provided herein that take advantage of the increase in the amount of PSMA on the surface of PSMA expressing cells as a result of folate restriction. Furthermore, as demonstrated herein, hormonal therapy administered to prostate cancer patients to deprive the prostate cancer cells of androgens also results in an increase in the amount of PSMA on the surface of PSMA expressing cells. After serum levels of testosterone reach castrate levels (≤50 ng/mL), the amount of PSMA on the surface of PSMA expressing cells increases by about nine -fold. Methods of treating cancer are provided herein that take advantage of the increase in the amount of PSMA on the surface of PSMA expressing cells as a result of hormonal therapy. Treatment with an antibody or antigen binding fragment thereof that is capable of binding to PSMA and inhibiting PSMA enzymatic activity can be combined with other therapies to treat cancers such as prostate cancer and other tumors that have PSMA expressing cells in the vascular endothelium of the tumor.

### METHODS FOR TREATING CANCER, FOLATE CONTROL

Provided herein are methods and compositions for treating cancer in a patient. In some embodiments of treating cancer, the method comprises administering an antibody or antigen binding fragment thereof to the patient, wherein the antibody or antigen binding fragment thereof is capable of binding to an extracellular domain of PSMA and inhibiting enzymatic activity of the PSMA, and restricting intake of folate by the patient. In some embodiments, intake of folate containing dietary supplements by the patient is proscribed (prohibited). In other embodiments, intake of folate by the patient is restricted to 400 µg per day or less, or such that the serum level of folate in the patient is 10 nmol/L or less, or such that the red blood cell (RBC) folate level in the patient is 300 nmol/L or less. In some embodiments, intake of folate by the patient is restricted to 300, 200, 100, 50, 5 µg per day or less. The levels of folate described herein are based on a chemiluminescent immunoassay and may vary somewhat when determined by other techniques. One of ordinary skill in the art would be able to compare folate levels determined using other techniques with the levels described herein.

Restriction of folate intake can include restricting the intake of membrane permeable forms of folate such as folic acid (found in dietary vitamin supplements), or restriction of intake membrane impermeable forms of folate (folylpolyglutamate) that are found naturally in certain foods, or combinations thereof. Preferably, folate is restricted such that intra-tumoral folate levels are decreased.

In other embodiments, the method of treating cancer in a patient comprises administering an antibody or antigen binding fragment thereof to a patient, wherein the antibody or antigen binding fragment thereof is capable of binding to an extracellular domain of PSMA and inhibiting folate hydrolase activity of the PSMA in combination with monitoring serum folate levels of the patient. In some embodiments, the intake of folate by the patient can be increased or decreased depending on the measurement of the serum folate levels of the patient.

In some embodiments, the method of treating cancer in a patient comprises administering an antibody or antigen binding fragment thereof to a patient, wherein the antibody or antigen binding fragment thereof is capable of binding to an extracellular domain of PSMA and inhibiting enzymatic activity of the PSMA and measuring serum levels of the antibody or antigen binding fragment thereof in order, for example, to maintain optimal levels of enzymatic inhibition. In other embodiments, the method comprises administering the antibody or antigen binding fragment thereof as described above and measuring residual PSMA activity of PSMA expressing cells of the patient in order, for example, to maintain optimal levels of enzymatic inhibition. In some embodiments, the amount of antibody or antigen binding fragment thereof administered to the patient can be increased or decreased depending on the measurement of PSMA activity of PSMA expressing cells of the patient.

### METHODS FOR TREATING CANCER, PSMA UPREGULATION

Exposing PSMA-expressing cells to reduced levels of folate results in an increase in surface levels of PSMA on PSMA expressing cells. This increase peaks about 3 to 4 weeks after exposure to reduced levels of forms of folate has begun or in shorter time periods if the prior intracellular folate stores are lower. As a result, PSMA increased by ten fold about three to four weeks after exposure to reduced levels of folate has begun. Therefore, methods of treating cancer are provided that take advantage of the increase in surface levels of PSMA on PSMA expressing cells as a result of exposure to reduced levels of membrane permeable forms of folate.

In some embodiments, the method of treating cancer comprises administering a first antibody or antigen binding fragment thereof to the patient wherein the first antibody or antigen binding fragment thereof is capable of binding to an extracellular domain of PSMA and inhibiting the enzymatic activity of the PSMA, restricting intake of folate by the patient, and administering a second antibody or antigen binding fragment thereof that is capable of binding to an extracellular domain of PSMA to the patient. In some embodiments, intake of folate by the patient is restricted such that folate intake is 400 µg per day or less, or such that the serum level of folate in the patient is 10 nmol/L or less, or such that the red blood cell (RBC) folate level in the patient is 300 mnol/L or less. In some embodiments, the second antibody or antigen binding fragment thereof is capable of binding to a different epitope of the extracellular domain of PSMA. In some embodiments, the second antibody or antigen binding fragment thereof is conjugated to a cytotoxic and/or radioisotopic agent or is capable of eliciting a secondary immune response. In some embodiments, the first dose of the second antibody or antigen binding fragment thereof is administered two to four weeks after intake of folate has been restricted. In some embodiments, the first dose of the second antibody or antigen binding fragment thereof is administered about 2, 3, or 4 weeks after intake of folate has been restricted.

In other embodiments, the method of treating cancer comprises restricting intake of folate by the patient and administering an antibody or antigen binding fragment thereof to the patient wherein the antibody or antigen binding fragment thereof is capable of binding to an extracellular domain of PSMA, wherein the first dose of the antibody or antigen binding fragment thereof is administered two to four weeks after intake of folate has been restricted. In some embodiments, the first dose of the antibody or antigen binding fragment thereof is administered about 2, 3, or 4 weeks after intake of folate has been restricted. In some embodiments, the amount of PSMA present on PSMA expressing cells of the patient can be measured to determine whether the level of PSMA on the surface of PSMA expressing cells has increased. The first does of the antibody or antigen binding fragment thereof can be administered after it is determined that the level of PSMA on the surface of PSMA expressing cells has increased.

Surface levels of PSMA on PSMA expressing cells increases as a result of hormonal therapy designed to remove androgens that fuel prostate cancer growth. This increase begins after testosterone levels decrease and/or reach castrate levels (about 50 ng/mL or less) and peaks two to three weeks later. As a result, PSMA expression on the surface of PSMA expressing cells increases by about nine-fold. Therefore, methods of treating prostate cancer are provided that take advantage of the increase in surface levels of PSMA on PSMA expressing cells as a result of hormonal therapy. Furthermore, methods of treating prostate cancer are provided that combine the benefits of folate control and hormonal therapy.

In some embodiments, the method of treating prostate cancer comprises administering hormonal therapy to the patient and administering an antibody or antigen binding fragment thereof to a patient, wherein the antibody or antigen binding fragment thereof is capable of binding to an extracellular domain of PSMA. In some embodiments, the first dose of the antibody or antigen binding fragment thereof is administered to the patient two to three weeks after serum testosterone levels of the patient have reached castrate levels (50 ng/mL or less).

Anti-PSMA antibody treatment can be timed to coincide with the timing of increased surface expression of PSMA and thereby deliver an agent whose efficacy is a function of the surface density of PSMA as early as possible during the progression of the disease. For example, where the antibody or antigen binding fragment thereof is conjugated to a radioisotope, is it desirable to deliver the highest amounts of radiation possible to the tumor cell. However, because of the inherent marrow toxicity of radioisotope based treatments, the number of doses that can be given is limited. Delivery of cytotoxin-conjugated antibody or antigen binding fragment thereof will also benefit from timing the administration to coincide with increased expression of PSMA at the cell surface. However, because is possible to administer such treatments more often than radioimmunotherapy, cytotoxic antibody therapy is somewhat less dependent on the up-regulation of PSMA. In both cases, the dose of isotope and cytotoxin delivered into the tumor cell is a direct function of the density of PSMA at the cell surface and, therefore, coordinating the timing of anti-PSMA therapy with the upregulation of PSMA expression is beneficial.

Furthermore, as described herein, Lutetium-177 and other short-range alpha or beta-emitting isotopes appear to be more effective as a radiolabel for antibodies or antigen binding fragments thereof that are capable of binding to an extracellular domain of PSMA when used to treat patients with earlier stage, non-bulky prostate cancer. As described herein, patients with an earlier stage, non-bulky prostate cancer typically have an abnormally elevated serum Prostate Specific Antigen (PSA) but lack evidence of cancer spread on imaging studies (e.g., higher volume soft tissue disease that is apparent on CT or MRI scans). An abnormally elevated PSA consists of a PSA greater then 0.1 ng/mL after a prior radical prostatectomy or greater than 0.5 ng/mL after prostatic radiotherapy or greater than 4.0 ng/mL in the absence of prior treatment to the prostate. In one embodiment, early stage prostate cancer comprises elevated and/or rising PSA levels and no evidence of soft tissue disease greater than 0.9 cm in diameter. Therefore, treatment regimens are provided that take advantage of the upregulation of PSMA in response to hormonal therapy and/or dietary folate restriction with or without inhibition of PSMA's folate hydrolase activity and the sensitivity of non-bulky prostate cancer to short range isotopes such as Lutetium-177.

In some embodiments, the method of treating prostate cancer comprises administering hormonal therapy to the patient, wherein the patient has been diagnosed with early stage, non-bulky prostate cancer and administering an antibody or antigen binding fragment thereof that is capable of binding to an extracellular domain of PSMA, wherein the antibody or antigen binding fragment thereof is conjugated to Lutetium-177 or other short range isotope. In some embodiments, the first dose of the antibody or antigen binding fragment thereof is administered three to four weeks after hormonal therapy is begun.

### METHODS OF MONITORING THERAPY

Also provided herein are methods of monitoring cancer therapy in a patient. In some embodiments, methods of monitoring cancer therapy comprise measuring blood levels of folate in the patient, wherein folate intake by the patient is being restricted and wherein the patient has received at least one dose of an antibody or antigen binding fragment thereof that is capable of binding to an extracellular domain of PSMA and inhibiting the enzymatic activity of-PSMA.

In other embodiments, methods of monitoring cancer therapy in a patient comprise measuring PSMA activity of PSMA expressing cells of a patient, wherein folate intake by the patient is being restricted and wherein the patient has received at least one dose of an antibody or antigen binding fragment thereof that is capable of binding to an extracellular domain of PSMA and inhibiting enzymatic activity of the PSMA. In some embodiments, folate intake is restricted such that folate intake is 400 µg per day or less, or such that the serum level of folate in the patient is 10 nmol/L or less, or such that the red blood cell (RBC) folate level in the patient is 300 nmol/L or less.

### KITS

Also provided herein are kits for treating cancer. In some embodiments, the kits comprise an antibody or antigen binding fragment thereof that is capable of binding to an extracellular domain of PSMA and inhibiting enzymatic activity of the PSMA, and instructions for restricting intake of folate by the patent. In some embodiments, the antibody or antigen binding fragment there of is conjugated to a cytotoxic agent or is capable of eliciting an antibody-dependent cellular cytotoxic response.

Also provided herein are kits for monitoring cancer therapy in a patient. In some embodiments, the kits comprise a tissue or blood collection apparatus, a PSMA activity detection reagent, and instructions for testing tissue or blood obtained from the patient using the PSMA activity detection reagent.

In other embodiments, the kits comprise a blood collection apparatus; a folate detection reagent, and instructions for testing red blood cells or serum obtained from the patient using the folate detection reagent. In some embodiments, the kits further comprise instructions for reducing folate intake by the patient based folate levels detected using the kit.

Suitable PSMA activity detection reagents include, for example, a PSMA substrate and/or one or more antibodies or antigen binding fragments thereof that are capable of binding to PSMA. In some embodiments, the PSMA substrate or at least one of the antibodies or antigen binding fragments thereof is immobilized on a solid surface. In some embodiments, the antibody or antigen binding fragment thereof is labeled with a detectable label. Where additional antibodies or antigen binding fragments are present, such additional antibodies or antigen binding fragments thereof can be labeled with detectable label that is distinguishable from the label of the first antibody or antigen binding fragment thereof. Suitable detectable labels include, for example, fluorescent moieties, radioisotopes, or enzyme labels such as horseradish peroxidase or alkaline phosphatase. The kit can also include instructions for testing PSMA activity.

In some embodiments, the kit comprises a PSMA detection reagent, and instructions for testing tissue or blood obtained from the patient using the PSMA detection reagent. In one embodiment, a tissue, blood, serum or plasma sample from a patient is reacted with a PSMA enzyme substrate under conditions suitable to allow the PSMA to act upon the substrate and produce a detectable product in proportion to the amount of PSMA activity present in the sample.

### DOSING AND TREATMENT REGIMEN

The antibodies or antigen binding fragments thereof described herein can be administered to a patient (also referred to herein as a subject) in single or multiple doses to treat or prevent a prostatic or cancerous disorder. The doses of antibody or antigen binding fragment thereof administered to a subject can be chosen in accordance with different parameters, in particular in accordance with the mode of administration used and the state of the subject. Other factors include the desired period of treatment, and whether other forms of treatment are being co-administered or used in conjunction with the antibody or antigen binding fragment thereof. As described above, the level of antibody or antigen binding fragment thereof or PSMA enzymatic activity can be monitored in the patient and the dose of antibody or antigen binding fragment thereof can be adjusted according to the detected levels.

In general, a dose of antibody or antigen binding fragment thereof can range from about 1 to about 1000 mg. In some embodiments, the antibody or antigen binding fragment thereof is administered in amount sufficient to achieve maximal inhibition of PSMA enzymatic activity. In some embodiments, an antibody or antigen binding fragment thereof is an antibody is administered to the patient in sufficient amount to achieve a serum concentration of at least about 5 ug/mL of antibody in the subject. In some embodiments, the antibody or antigen binding fragment thereof is administered in sufficient amount to achieve a serum concentration of 10, 25, 50, 100, or 200 ug/mL. In some embodiments, an antibody or antigen binding fragment thereof is an antigen binding fragment of an antibody, such as a (Fab')₂ and is administered to the patient in sufficient amount to achieve a serum concentration of at least about 3.3 ng/mL of the antigen binding fragment thereof in the subject. In some embodiments the (Fab')₂ is administered to achieve a serum concentration of 6.6, 10, 20, 40, or 80 ng/mL. In some embodiments, the antibody or antigen fragment thereof is administered in sufficient amount to achieve a sustained serum concentration of the desired amount. The antibody or antigen binding fragment thereof can be administered to such that the serum level is sustained for the desired period of time. The desired serum level can be based on the amount of antibody or antigen binding fragment thereof that can be measured in a sample of blood, serum or plasma or can be based on the level of inhibition of PSMA activity. In some embodiments, the antibody or antigen binding fragment thereof is administered in sufficient amount to achieve greater than 50, 60, 70, 80, or 90% inhibition of PSMA activity of PSMA expressing cells of the patient. The dose of antibody or antigen binding fragment thereof can be adjusted by one or ordinary skill in the art based, for example on the size of the antibody or antigen binding fragment thereof, and the binding affinity of the antibody or antigen binding fragment thereof and PSMA. A suitable level of antibody or antigen binding fragment thereof in the serum can be maintained by way of repetitive dosing.

In some embodiments, serum trough and/or peak levels of antibody or antigen binding fragment thereof can be determined prior to administering the next dose of antibody or antigen binding fragment thereof. Serum trough and/or peak levels can be determined using standard techniques known in the art. Serum trough and/or peak levels can be used to adjust the prescribed dose of antibody or antigen binding fragment thereof to individual patients or groups of patients.

A variety of routes can be used to administer the antibody or antigen binding fragment thereof. The particular mode selected will depend upon the particular drug selected, the severity of the disease state being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, sublingual, topical, nasal, transdermal or parenteral routes. The term "parenteral" includes subcutaneous, intravenous, intramuscular, or infusion.

The antibody or antigen binding fragment thereof can be administered once, continuously, such as by continuous pump, or at periodic intervals. The periodic interval may be weekly, bi-weekly, or monthly. The dosing can occur over the period of one month, two months, three months or more to elicit an appropriate humoral and/or cellular immune response or to maintain a desired level of enzymatic inhibition of PSMA. Desired time intervals of multiple doses of a particular composition can be determined without undue experimentation by one skilled in the art. Other protocols for the administration of an antibody or antigen binding fragment thereof will be known to one of ordinary skill in the art, in which the dose amount, schedule of administration, sites of administration, mode of administration and the like vary from the foregoing.

In some embodiments of the methods and kits provided herein, one or more of the antibodies or antigen binding fragments thereof described herein is used in a non-derivatized or unconjugated form (also referred to herein as "naked" or "unlabeled").

In other embodiments of the methods and kits provided herein, one or more of the antibodies or antigen binding fragments thereof described herein is conjugated to an agent. PSMA is normally internalized from the cell membrane into the cell. Thus, the antibody or antigen binding fragment thereof that is capable of binding to the extracellular domain of PSMA is capable of being internalized with PSMA thereby permitting delivery of an agent conjugated to the antibody. The agent can be, for example, a labeling agent, a cytotoxic agent, a nano-particle or a viral particle (e.g., a viral particle containing genes that encode cytotoxic agents, e.g., apoptosis-promoting factors).

In some embodiments, the antibody or antigen-binding fragment thereof can be conjugated or linked to another molecular entity, typically a label or a therapeutic (e.g., a cytotoxic or cytostatic) agent. The antibody or antigen-binding fragment thereof can be functionally linked, e.g., by chemical coupling, genetic fusion, non-covalent association or otherwise, to one or more other molecular entities.

In some embodiments, the label is, for example, a fluorescent label, a biologically active enzyme label, a radioisotope (e.g., a radioactive ion), a nuclear magnetic resonance active label, a luminescent label, or a chromophore. In some embodiments, the therapeutic agent is, for example, cytotoxic moiety such as a therapeutic drug, a radioisotope, molecules of plant, fungal, or bacterial origin, or biological proteins (e.g., protein toxins) or particles (e.g., nano-particles or recombinant viral particles, e.g., via a viral coat protein), or mixtures thereof. The therapeutic agent can be an intracellularly active drug or other agent, such as short-range radiation emitters, including, for example, short-range, high-energy α-emitters, as described herein. Suitable radioisotope include an α-, β-, or γ-emitter, or β- and γ-emitter. Radioisotopes useful as therapeutic agents include yttrium (⁹⁰Y), lutetium (¹⁷⁷Lu), actinium (²²⁵Ac), astatine (²¹¹At), rhenium (¹⁸⁶Re), bismuth (²¹²Bi or ²¹³Bi), and rhodium (¹⁸⁸Rh). Radioisotopes useful as labels, e.g., for use in diagnostics, include iodine (¹³¹I, ¹²⁴I or ¹²⁵I), indium (¹¹¹In), technetium (^{99m}Tc), phosphorus (³²P), carbon (¹⁴C), and tritium (³H), or one of the therapeutic isotopes listed above. In some embodiments, the antibody or antigen binding fragment thereof can be coupled to a molecule of plant or bacterial or fungal origin (or derivative thereof), e.g., a maytansinoid (e.g., maytansinol or the DM1 maytansinoid), a taxane, or a calicheamicin. The antibody or antigen-binding fragment thereof can also be linked to another antibody to form, e.g., a bispecific or a multispecific antibody.

In some embodiments, the antibody or antigen-binding fragment thereof is coupled, e.g., by covalent linkage, to a proteosome inhibitor or a topoisomerase inhibitor. [(1R)-3-methyl-1-[[(2S)-1-oxo-3-phenyl-2-[(3-mercaptoacetyl) amino]propyl]amino]butyl] Boronic acid is a suitable proteosome inhibitor. N,N'-bis[2-(9-methylphenazine-1-carboxamido)ethyl]-1,2-ethanediamine is a suitable topoisomerase inhibitor.

In some embodiments, the antibody or antigen-binding fragment thereof is used in combination with a small molecule or peptide inhibitor or aptamer inhibitor of PSMA activity. Suitable small molecule inhibitors of PSMA activity include, by way of example, quisqualate and 2-(phosphonomethyl)-pentanedioic acid (2-PMPA). The small molecule PSMA inhibitor may be linked to the antibody or antigen binding portion thereof or may be unlinked to the antibody or antigen binding portion thereof. Suitable peptide inhibitors of PSMA activity include, for example, WQPDTAHHWATL (SEQ ID NO. 1) and dimeric and/or multimeric forms thereof. Peptide inhibitors or aptamer inhibitors may or may not be linked to the antibody or antigen binding portion thereof. The methods provided herein allow the use of low doses of the small molecule inhibitor or peptide inhibitors or aptamer inhibitors of PSMA thereby minimizing the side effects of these agents at non-tumor sites such as the kidney proximal tubule, small bowel and/or brain.

In some embodiments, the antibody or antigen-binding fragment thereof is linked to a therapeutic agent as described herein via a linker, e.g., a cleavable linker or a non-cleavable linker. The use of a cleavable linker allows the release of the therapeutic agent into the intracellular cytoplasm upon internalization of the conjugated antibody or antigen-binding fragment thereof. A non-cleavable linker would allow release upon digestion of the antibody or antigen binding portion thereof or it could be used with an agent that does not require release from the antibody.

### COMBINATION WITH OTHER THERAPIES

In some embodiments of the methods of treating cancer, the antibody or antigen binding fragment thereof that is capable of binding to an extracellular domain of PSMA and inhibiting enzymatic activity can be used in combination with other therapies. In some embodiments, other therapies include administering to the subject a cytotoxic or chemotherapeutic agent. Exemplary cytotoxic agents include antimicrotubule agents, topoisomerase inhibitors, antimetabolites, mitotic inhibitors, alkylating agents, intercalating agents, agents capable of interfering with a signal transduction pathway, agents that promote apoptosis agents that interfere with folate metabolism and radiation. In some embodiments, the cytotoxic agent can be Taxol, taxotere, cytochalasin B, gramicidin D, ethidium bromide, emetine, mitomycin, etoposide, tenoposide, vincristine, vinblastine, colchicin, doxorubicin, daunorubicin, dihydroxy anthracin dione, methotrexate, mitoxantrone, mithramycin, actinomycin D, 1-dehydrotestosterone, glucocorticoids, procaine, tetracaine, lidocaine, propranolol, puromycin, a maytansinoid, e.g., maytansinol (see U.S. Pat. No. 5,208,020), CC-1065 (see U.S. Pat. Nos. 5,475,092, 5,585,499, 5,846,545) and/or analogs or homologs thereof. Suitable agents that interfere with folate metabolism (folate antagonists) include, for example, methotrexate, aminopterin, trimetrexate, lometrexol, pemetrexed, thymitaq and 5-fluorouracil. Chemotherapeutic agents also include, for example, inhibitors of PSMA activity.

In some embodiments, administering an antibody or antigen binding fragment thereof that is capable of binding to the extracellular domain and inhibiting enzymatic activity of the PSMA can be combined with a lower dose of the cytotoxic agent, thereby reducing the likelihood of undesirable side effects from the cytotoxic agent. In some embodiments, the cytotoxic agent is administered at 10%, 20%, 30%, 40%, 50%, 60%, 70%, or 80% of the standard dose that would normally be administered to a patient having a similar stage of cancer and similar physical characteristics such as, weight, height, age, and treatment history. Similarly, restricting folate intake allows the administration of a lower amount of cytotoxic agent when the therapies are used in combination.

The antibody or antigen binding fragment thereof that is capable of binding to an extracellular domain of PSMA and inhibiting enzymatic activity is administered in conjunction with a therapy that is more effective against actively proliferating cells. In some embodiments, the cycle of restricting intake of folate by the patient and administering the antibody or antigen binding fragment thereof is administered in alternating fashion with the cycle of administering the other therapy. In this manner, the effect of the other therapy is maximized because it is administered during windows of time that allow cell proliferation.

In other embodiments, the antibody or antigen binding fragment thereof that is capable of binding to an extracellular domain of PSMA and inhibiting enzymatic activity can be used in combination with surgical and/or radiation procedures. In yet other embodiments, the antibody or antigen binding fragment thereof that is capable of binding to an extracellular domain of PSMA and inhibiting folate hydrolase activity can be used in combination with immunomodulatory agents, e.g., IL-1, 2, 4, 6, or 12, or antagonists thereof, or interferon alpha or gamma, or cell growth factors such as GCSF and/or GM-CSF. The antibody or antigen binding fragment thereof that is capable of binding to an extracellular domain of PSMA and inhibiting folate hydrolase activity can also be administered with other agents given to reduce the side effects of cancer treatment including, e.g., one or more of a treatment which stimulates the production of red cells (e.g., erythropoietin (EPO)), a treatment which promoters bone formation or structure (e.g., biphosphonates (e.g., pamideonate disodium and/or zoledronate)), and a treatment for other side effects (e.g., acetaminophen and diphenyldramine hydrochloride).

Where the methods and compositions provided herein are used to treat patients having prostatic disorders, e.g., prostate cancer, the antibody or antigen binding fragment thereof that is capable of binding to an extracellular domain of PSMA and inhibiting folate hydrolase activity can be used in combination with existing therapeutic modalities, e.g., prostatectomy (partial or radical), radiation therapy, prostatic ablation therapy (e.g. hormonal therapy, cryosurgery, laser ablation, high intensity focused ultrasound, etc.), and cytotoxic chemotherapy as described above. Typically, hormonal therapy works to reduce the levels of androgens in a patient, and can involve administering a leuteinizing hormone-releasing hormone (LHRH) analog or agonist (e.g., Lupron®, Zoladex®, leuprolide, buserelin, or goserelin), as well as antagonists (e.g., Abarelix). Non-steroidal anti-androgens, e.g., flutamide, bicalutimade, or nilutamide, can also be used in hormonal therapy, as well as steroidal anti-androgens (e.g., cyproterone acetate or megastrol acetate), estrogens (e.g., diethylstilbestrol), surgical castration, secondary or tertiary hormonal manipulations (e.g., involving corticosteroids (e.g., hydrocortisone, prednisone, or dexamethasone), ketoconazole, abiraterone and/or aminogluthethimide), inhibitors of 5a-reductase (e.g., finasteride), herbal preparations, hypophysectomy, and adrenalectomy. Furthermore, hormonal therapy can be performed continuously, intermittently or using combinations of any of the above treatments, e.g., combined use of leuprolide and bicalutamide.

In some embodiments, the first dose of the antibody or antigen binding fragment thereof is administered two or more weeks after administering an LHRH antagonist such as abarelix. In some embodiments the first dose of the antibody or antigen binding fragment thereof is administered two or more weeks after administering an LHRH agonist in conjunction with an anti-androgen such as bicalutimide, flutamide, nilutimide.

The antibody or antigen-binding fragment thereof that is capable of binding to an extracellular domain of PSMA and inhibiting folate hydrolase activity can be used in combination with another antibody or antigen binding fragment thereof, that binds to, for example, PSMA (e.g., an extracellular portion of PSMA) or an antigen other than PSMA (e.g., PSCA (prostate stem cell antigen) or six transmembrane epithelial antigen of prostate (STEAP)). One or both of the antibodies or antigen-binding fragments thereof can be conjugated or unconjugated as described above. When both are conjugated, they can be conjugated with the same or different therapeutic agents or labels.

The antibody or antigen-binding fragment thereof that is capable of binding to an extracellular domain of PSMA and inhibiting folate hydrolase activity can be used in combination with other therapies or preventative treatments, such as anti-cancer vaccines.

### MEASURING FOLATE LEVELS

In some embodiments of methods of treating or monitoring the treatment of cancer, the method comprises measuring levels of folate in the patient, wherein the patient has been treated with an antibody or antigen binding fragment thereof that is capable of binding to an extracellular domain of PSMA and inhibiting folate hydrolase activity of the PSMA

PSMA enzymatic activity can be detected and/or measured using techniques known in the art including, for example, calorimetric, densitometric, spectrographic and chromatographic assays and imaging techniques (such as magnetic resonance spectroscopy (MRS), magnetic resonance imaging (MRI), single-photon emission computed tomography (SPECT) and positron emission tomography (PET)) together with detectable substrates for NAALADase (also referred to herein as a PSMA substrate) or detectable PSMA binding molecules.

Measurement of folate levels in the patient can include measuring serum or plasma folate levels, red cell folate, or both, using standard techniques such as chemiluminescent immunoassay, radioimmunoassay or microbiologic methods. In some embodiments, the level of serum folate is measured as described, for example by Waxman and Schreiber, Blood, 42(2):281-290 (1972). Serum levels of folate are normally within the range of 7-40 nmol/L, depending on the age of the patient and method used. Stores of folate in the body can be depleted in 3 to 6 months of low folate intake by the patient, and a possible effect of restricted folate intake by the patient is folate deficiency anemia. A diagnosis of folate deficiency anemia is confirmed in part by a low serum folate level (< 2 ng/mL (< 5 nmol/L)) or a low RBC folate level (< 100 ng/mL (< 227 nmol/L)). Therefore, in some embodiments, the patient's serum or RBCs can be monitored for folate levels, and the intake of folate can be adjusted to maintain a serum folate level of about 5 nmol/L. In other embodiments, the folate restriction can be terminated after a certain period of time, such as one, two, three, four, five, or six months. In still other embodiments, the folate restriction can be prescribed such that intake is restricted for a certain period of time and then unrestricted for a certain period of time and the two periods of time can be alternated.

Folate levels in the patient can be increased or decreased as necessary by altering the intake of folate by the patient. For example, the patient can be prescribed a diet that restricts the amount of folic acid or other membrane permeable forms of folate that can be consumed. Folic acid (pteroglutamic acid) is found, for example, in dietary supplements in green leafy vegetables, whole grain, food products made with fortified flour and liver. In addition, or alternatively, if the patient is receiving intravenous fluids, the fluids can be restricted in the amount of folic acid present. In addition, or in the alternative, the patient can be prescribed a diet that restricts or prohibits the amount of foods rich in naturally occurring forms of folate. For example, food such as romaine lettuce, spinach, asparagus, turnip greens, mustard greens, calfs liver, parsley, collard greens, broccoli, cauliflower, beets, and lentils are rich sources of folate. The patient can be instructed to avoid consuming such foods or to consume only limited quantities of such foods. The patient can be provided with a list of foods that are rich in folate.

In some embodiments, the level of serum or RBC folate of the patient may drop to a level that is too low, for example below 5 nmol/L in the case of serum folate. Therefore, in some embodiments, folate can be administered to the patient to raise the serum level to 5 nmol/L. The folate can be administered by any suitable technique, such as by i.v., or orally. The patient can be prescribed a suitable supplement that contains a dose of folic acid sufficient to raise serum folate to 5 nmol/L but that would not raise the serum folate, for example above 10 nmol/L. Alternatively, the patient can be instructed to consume or eat a prescribed quantity of food rich in polyglutamylfolate (folylpolyglutamate).

### MONITORING THERAPY, MEASURING PSMA

In some embodiments, methods of detecting and/or measuring PSMA comprise measuring and/or detecting PSMA enzymatic activity (also referred to herein as PSMA activity). In some embodiments, measuring PSMA activity comprises assaying PSMA activity in a sample of tissue or bodily fluid of a patient. Any suitable assay for detecting the level of enzyme activity in a sample can be used. For example, the hydrolysis of a detectable or labeled substrate of PSMA can be used. A substrate of PSMA is also referred to herein as an enzymatic substrate. The sample oftissue or bodily fluid to be tested can be brought into contact with the PSMA substrate, resulting in a quantity of detectable or labeled metabolite which can be detected using suitable separation and/or detection methods for that metabolite. The quantity of labeled metabolite from the sample can be compared to at least one reference or control wherein the reference or control has a known quantity of PSMA. Any suitable control or reference can be used that allows a quantitative or qualitative assessment of the amount PSMA in the sample. For example, a positive control or reference can be represented by a quantity of labeled metabolite from tissue or bodily fluid which is indicative of an amount of enzymatically active PSMA in prostate cancer cells or the neovasculature or other PSMA expressing tumors. A negative control or reference can represent a quantity of labeled metabolite from tissue or bodily fluid which is indicative of the absence of active PSMA or the presence of low levels of active PSMA. Comparing the level of the detectable or labeled metabolite produced by the sample and the control or reference indicates the level of active PSMA in the sample. The PSMA activity can be a quantitative value of a detectable metabolite of PSMA activity. In other embodiments, the PSMA activity is a qualitative value of a detectable metabolite of PSMA compared to a standard or control sample.

Suitable PSMA substrates include N-Acetyl Aspartyl Glutamate (NAAG), folate polyglutamate, methotrexate tri-gamma glutamate, methotrexate di-gamma glutamate, pteroylpentaglutamate and derivatives thereof. The substrates can be labeled, for example, with a radioactive marker, chemiluminescent marker, enzymatic marker, chromogenic marker, or other detectable marker. Suitable methods for detecting PSMA activity are described, for example, in US 5,981,209 at col. 6, line 60 through col. 7, line 40 and at col. 8, line 63 through col. 9 line 20, and, for example, in Clin. Cancer Res. 2:1445-1451 (1996); Urology 49:104-112 (1997), the teachings of which are incorporated herein by reference. PSMA activity can also be measured in vitro using known methods in the art. For example as described in paragraphs [0281]-[0283] of US published patent application 2006/0009525.

Suitable samples can be any bodily tissue or fluid that is expected or suspected of containing PSMA or PSMA expressing cells. In some embodiments, the sample can be treated to solubilize and/or release the PSMA from the cellular membrane. In some embodiments, the sample can be treated to purify or at least partially purify the PSMA away from the sample milieu. Preferably, any treatment of the sample is such that any enzymatically active PSMA retains enzyme activity and any enzymatically inactive PSMA remains enzymatically inactive.

In some embodiments, the amount of PSMA can be measured *in vivo* using known imaging techniques, as described above using a suitable PSMA substrate or a detectable PSMA binding molecule. The suitable PSMA binding molecule can be, for example, an antibody or antigen binding fragment thereof that is capable of binding to PSMA. In some embodiments, the antibody or antigen binding fragment thereof is capable of binding the extracellular domain of PSMA. Suitable PSMA binding molecules may be labeled with a detectable marker using techniques known in the art. Useful detectable markers include, without limitation, enzymatic substrates and imaging reagents. Examples of imaging reagents include radiolabels such as ¹³¹I, ¹¹¹In, ¹²³I, ⁹⁹Tc, ³²P, ¹²⁵I, ³H and ¹⁴C; fluorescent labels such as fluorescein and rhodamine; and chemiluminescent molecules such as luciferin. Suitable enzymatic reagents are described above.

In some embodiments, the binding of the antibody or antigen binding fragment thereof to PSMA inhibits the ability of the PSMA to form dimers. Therefore, "PSMA activity" can be measured indirectly by detecting or measuring the amount of PSMA dimers using, for example, a PSMA binding molecule. Suitable PSMA binding molecules include, for example, antibodies or antigen binding fragments thereof that are capable of binding PSMA. The PSMA binding molecule can be labeled with a detectable marker as described above. PSMA dimers can be detected or measured, for example, using PSMA binding molecules that are capable of binding to dimeric PSMA and monomeric PSMA or PSMA binding molecules that are capable of specifically binding to dimeric PSMA but not monomeric PSMA. Where the PSMA binding molecule is specific for dimeric PSMA measuring dimeric PSMA comprises contacting a sample or portion thereof with the binding molecule under conditions suitable for the binding molecule to bind any dimeric PSMA present and determining the level of bound PSMA binding molecule.

Where the PSMA binding molecule is capable of binding monomeric or dimeric PSMA, measuring PSMA dimers can comprise, for example, contacting a sample or portion thereof with the binding molecule under conditions suitable for the antibody or antigen binding fragment thereof to bind any PSMA (monomeric or dimeric) present and determining the level of bound binding molecule. A sample or portion thereof is contacted with a PSMA binding molecule that is capable of binding monomeric PSMA but not dimeric PSMA, under conditions suitable for the binding molecule to bind any monomeric PSMA present and determining the level of bound binding molecule. The level of binding molecule capable of binding to dimeric and monomeric PSMA is compared to the level of binding molecule capable of binding to monomeric PSMA is compared, to determine the level dimeric PSMA.

Also provided herein are methods of monitoring cancer therapy in a patient. In some embodiments, the method comprises obtaining PSMA expressing cells from a patient that has been treated with an antibody or antigen binding fragment thereof that is capable of binding to an extracellular domain of PSMA and inhibiting folate hydrolase activity of the PSMA and measuring PSMA activity of the cells.

PSMA expressing cells can be obtained as part of or from any suitable source of cells from the patient that is thought to contain PSMA expressing cells. For example the source of cells can be biopsy material from a solid tumor that includes vascular endothelial cells. The source of cells can be biopsy material from a primary prostate tumor or from tumors derived from secondary sites. The source of cells can be blood which is thought to include circulating prostate cancer cells. The cells can be obtained from the patient using standard techniques and preserved such that PSMA, if present can be detected.

PSMA activity can be measured indirectly by measuring the amount of dimeric PSMA present on the cells. PSMA exists as a dimeric molecule and a monomeric molecule. The dimeric form of PSMA has enzymatic activity, but the monomeric form does not. Detectably labeled antibodies or antigen binding fragments thereof that are capable of binding PSMA can be used to measure the amount of dimeric PSMA present on the cells. For example, a detectably labeled antibody or antigen binding fragment thereof that specifically recognizes the dimeric form can be used to detect PSMA on the cells. The cells can be contacted with the detectably labeled antibody or antigen binding fragment thereof under conditions suitable for the antibody or antigen binding fragment thereof to bind any dimeric PSMA present and determining levels of bound antibody or antigen binding fragment thereof.

In other embodiments, methods of treating cancer and methods of monitoring the treatment of cancer comprise measuring the level of anti-PSMA antibody or antigen binding fragment thereof in the serum. In some embodiments, the anti-PSMA antibody is capable of binding the extracellular domain of PSMA and inhibiting PSMA enzyme activity. Serum levels of anti-PSMA antibodies or antigen binding fragments thereof can be measured using standard techniques in the art, such as Enzyme Linked Immunosorbant Assay (ELIZA) using immobilized PSMA Serum is obtained from the patient using standard techniques in the art. The serum can be diluted by a desired factor and exposed to PSMA that has been immobilized on a solid support under conditions suitable to allow any anti-PSMA antibody or antigen binding fragment thereof to bind the immobilized PSMA. Unbound antibody or antigen binding fragment thereof is washed away and bound antibody or antigen binding fragment thereof can be detected using a detectably labeled antibody that is capable of binding the anti-PSMA antibody or antigen binding fragment thereof. In other embodiments, the level of anti-PSMA antibody or antigen binding fragment thereof can be measured by testing the ability of serum obtained from the patient to inhibit PSMA activity of PSMA expressing cells, such as LNCaP cells.

As described above, the amount of antibody or antigen binding fragment thereof administered to the patient can be adjusted based on the amount of PSMA activity or the serum level of antibody or antigen binding fragment thereof detected.

Also provided herein are kits that can be used in the methods for monitoring cancer therapy in a patient. In some embodiments, the kit comprises a tissue or blood collection apparatus, a PSMA detection reagent, and instructions for testing tissue or blood obtained from the patient using the PSMA detection reagent.

In some embodiments, the kit comprises a tissue or blood collection apparatus and at least one antibody or antigen fragment thereof that is capable of binding PSMA and instructions for testing the tissue, blood, or serum obtained from the patient using the at least one antibody or antigen binding fragment thereof. In one embodiment, a tissue, blood, or serum sample from a patient is from a patient is reacted with a solid phase reagent having surface-bound PSMA. After the PSMA is allowed to bind any specific antibody or antigen binding fragment thereof present in the serum, the unbound serum components are removed by washing. Detectably labeled antibody that is capable of specifically binding the antibody or antigen binding fragment thereof is added, unbound detectably labeled antibody is removed by washing, and the bound, labeled antibody is detected. Where the label of the detectably labeled antibody or antigen binding fragment thereof is an enzyme, a substrate for the enzyme is incubated with the solid phase under conditions suitable to allow the enzyme to act upon the substrate and produce a detectable product in proportion to the amount of bound antibody or antigen binding fragment thereof on the solid support. Typically, the reporter is an enzyme which is detected by incubating the solid phase in the presence of a suitable fluorometric, luminescent, or calorimetric substrate.

The solid surface having PSMA bound thereto can be prepared by known techniques for attaching protein material to solid support material, such as polymeric beads, dip sticks, 96-well plates or filter material. These attachment methods generally include non-specific adsorption of the protein to the support or covalent attachment of the protein, typically through a free amine group, to a chemically reactive group on the solid support, such as an activated carboxyl, hydroxyl, or aldehyde group. Alternatively, streptavidin coated plates can be used in conjunction with biotinylated antigen(s).

### TYPES OF CANCER

The methods of treating cancer provided herein can be used to treat any cancer that comprises at least some cells that express PSMA on their cell surfaces. In humans, PSMA is expressed on the surface of normal, benign hyperplastic epithelial cells (e.g., benign prostate secretory-acinar epithelium), cancerous prostate epithelial cells (e.g., prostatic intraepithelial neoplasia and prostatic adenocarcinoma), and vascular endothelial cells proximate of certain cancerous cells. Such cancerous cells include (but are not limited to), for example, renal, urothelial (e.g., bladder), testicular, colon, rectal, lung (e.g., non-small cell lung carcinoma), breast, liver, neural (e.g., neuroendocrine), glial (e.g., glioblastoma), pancreatic (e.g., pancreatic duct), melanoma (e.g., malignant melanoma), or soft tissue sarcoma cancerous cells.

Examples of prostatic disorders that can be treated or prevented include, but are not limited to, genitourinary inflammation (e.g., inflammation of smooth muscle cells) as in prostatitis; benign enlargement, for example, nodular hyperplasia (benign prostatic hypertrophy or hyperplasia); and cancer, e.g., adenocarcinoma or carcinoma, of the prostate and/or testicular tumors, including recurrent prostate cancer. "Recurrence" or "recurrent" prostate cancer, refers to an increase in PSA levels after an anti-cancer treatment (e.g., prostatectomy or radiation) to greater than 0.4 ng/dL in two consecutive tests spaced by a one month period. Cancer recurrence can occur over a short period of time from the anti-cancer treatment, e.g., a few months after treatment, or can occur several years after an anti-cancer treatment. For example, in prostate cancer patients, recurrence can happen several years after an anti-cancer treatment, e.g., up to 4, 5, 6, 7, 8, 9, 10, 12, 14, 15 years after treatment. Recurrence can be classified as "local recurrence" or "distant recurrence". "Local recurrence" refers to cancers which recur in tissue or organs adjacent to or proximate to the cancerous tissue or organ. For example, in subjects having prostate cancer, local recurrence can occur in tissue next to the prostate, in the seminal vesicles, the surrounding lymph nodes in the pelvis, the muscles next to the prostate, and the rectum and/or walls of the pelvis. "Distant recurrence" refers to cancers which recur distant from the cancerous tissue or organ. For example, in subjects having prostate cancer, distant recurrence includes cancers which spread to the bones or other organs.

The term "cancer" includes all types of cancerous growths or oncogenic processes, metastatic tissues or malignantly transformed cells, tissues, or organs, irrespective of histopathologic type or stage of invasiveness, where the cancerous cells or cells proximate to the cancerous cells (such as the vascular endothelial cells) express PSMA on their cell surface.

Examples of non-prostatic cancerous disorders include, but are not limited to, solid tumors, soft tissue tumors, and metastatic lesions. Examples of solid tumors include malignancies, e.g., sarcomas, adenocarcinomas, and carcinomas, of the various organ systems, such as those affecting lung, breast, lymphoid, gastrointestinal (e.g., colon), and genitourinary tract (e.g., renal, urothelial cells), pharynx. Adenocarcinomas include malignancies such as most colon cancers, rectal cancer, renal-cell carcinoma, liver cancer, non-small cell carcinoma of the lung, cancer of the small intestine and cancer of the esophagus. Metastatic lesions of the aforementioned cancers can also be treated or prevented using the methods and compositions provided herein.

The methods and compositions provided herein can be useful in treating malignancies of the various organ systems, such as those affecting lung, breast, lymphoid, gastrointestinal (e.g., colon), bladder, genitourinary tract (e.g., prostate), pharynx, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

### SUITABLE ANTIBODIES

The term "antibody" includes a protein comprising at least one, and preferably two, immunoglobulin heavy (H) chain variable regions (abbreviated herein as VH), and at least one and preferably two immunoglobulin light (L) chain variable regions (abbreviated herein as VL that is capable of specifically binding to a given antigen. As used herein, "specific binding" refers to the property of the antibody to bind to an antigen, e.g., PSMA, with an affinity of at least 1x10⁷ M⁻¹. In some embodiments, specific binding refers to the ability to bind to PSMA, e.g., human PSMA protein, with an affinity that is at least two-fold, 50-fold, 100-fold, 1000-fold, or more than its affinity for binding to an antigen other than PSMA (e.g., BSA, casein).

The VH and VL regions can be further subdivided into regions of hypervariability, termed "complementarity determining regions" ("CDR"), interspersed with regions that are more conserved, termed "framework regions" (FR). The extent of the framework region and CDRs has been precisely defined (see, Kabat, E. A., et al. (1991) Sequences of proteins of Immunological Interest, Fifth Edition, U.S. Department of Health and Human Services, NIH Publication No. 91-3242, and Chothia, C. et al. (1987) J. Mol. Biol. 196:901-917). In some embodiments, each VH and VL is composed of three CDRs and four FRs, arranged from amino-terminus to carboxy-terminus in the following order: FR1, CDR1, FR2, CDR2, FR3, CDR3, FR4.

The VH or VL chain of the antibody can further include all or part of immunoglobulin heavy or light chain constant regions. In one embodiment, the antibody is a tetramer of two immunoglobulin heavy chains and two immunoglobulin light chains. In some embodiments, the heavy and light chains are inter-connected by, e.g., disulfide bonds. In some embodiments, the heavy chain constant region is comprised of three domains, CH1, CH2 and CH3. In some embodiments, the light chain constant region is comprised of one domain, CL. The variable region of the heavy and light chains contains a binding domain that interacts with an antigen, e.g., the extracellular portion of PSMA or portion thereof. In some embodiments, the constant regions of the antibodies mediate the binding of the antibody to host tissues or factors, including various cells of the immune system (e.g., effector cells) and the first component (Clq) of the classical complement system. In this manner, the antibody can elicit an antibody-dependent cellular cytotoxic response and/or complement mediated cytotoxicity. The term "antibody" includes intact immunoglobulins of types IgA, IgG, IgE, IgD, IgM (as well as subtypes thereof), wherein the light chains of the immunoglobulin may be of types kappa or lambda.

The term "antigen binding fragment thereof" includes any portion of an antibody that specifically binds to the antigen (such as PSMA or an extracellular portion of PSMA). For example, an antigen-binding fragment of an antibody includes molecules in which one or more immunoglobulin chains is not full length but which is capable of specifically binding to the antigen. Examples of binding fragments encompassed within the term "antigen binding fragment thereof" include, for example, (i) a Fab fragment, e.g., a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) having sufficient framework capable of specifically binding to the antigen, e.g., an antigen binding portion of a variable region. An antigen binding portion of a light chain variable region and an antigen binding portion of a heavy chain variable region, e.g., the two domains of the Fv fragment, VL and VH, can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding fragment thereof". These antibody fragments are obtained using conventional techniques known to those of ordinary skill in the art, and the fragments are screened for binding ability in the same manner as are intact antibodies.

Many types of antibodies, or antigen binding fragments thereof, are useful in the methods and compositions provided herein. The antibodies can be of the various isotypes, including: IgG (e.g., IgG1, IgG2, IgG3, IgG4), IgM, IgA1, IgA2, IgD, or IgE. Preferably, the antibody is an IgG isotype, e.g., IgG1. The antibody molecules can be full-length (e.g., an IgG1 or IgG4 antibody) or can include only an antigen-binding fragment (e.g., a Fab, F(ab')₂, Fv or a single chain Fv fragment). These include monoclonal antibodies, recombinant antibodies, chimeric antibodies, humanized antibodies, deimmunized antibodies, and human antibodies, as well as antigen-binding fragments of the foregoing. Preferably, the monoclonal antibodies or antigen binding fragments thereof bind to the extracellular domain of PSMA or portion thereof (e.g., an epitope of PSMA located outside of a cell). Examples of preferred monoclonal antibodies that are capable of binding PSMA and are capable of inhibiting PSMA enzymatic activity include, but are not limited to, J415, which is produced by the hybridoma cell line having an ATCC Accession Number HB-12101.

The antibody or antigen binding fragment thereof can be humanized by methods known in the art. Once the murine antibodies are obtained, the variable regions can be sequenced. The location of the CDRs and framework residues can be determined (see, Kabat, E. A., et al. (1991) Sequences of Proteins of Imnunological Interest, Fifth Edition, U.S. Department of Health and Human Services, NIH Publication No. 91-3242, and Chothia, C. et al. (1987) J. Mol. Biol. 196:901-917). The light and heavy chain variable regions can, optionally, be ligated to corresponding constant regions.

The antibody or antigen binding fragment thereof may also be modified to delete specific human T cell epitopes (also referred to herein as "deimmunized"). Methods suitable for deimmunizing antibodies are disclosed, for example, in WO 98/52976 and WO 00/34317. Briefly, the heavy and light chain variable regions of the antibody or antigen binding fragment thereof (for example a murine antibody or antigen binding fragment thereof) can be analyzed for peptides that bind to MHC Class II; these peptides represent potential T-cell epitopes (as defined in WO 98/52976 and WO 00/34317). For detection of potential T-cell epitopes, a computer modeling approach can be applied, and in addition a database of human MHC class II binding peptides can be searched for motifs present in the murine V_{H} and V_{L} sequences. These motifs bind to any of the 18 major MHC class II DR allotypes, and thus constitute potential T cell epitopes. Any potential T-cell epitopes detected can be eliminated by substituting amino acid residues in the variable regions or by single amino acid substitutions. As far as possible conservative substitutions are made, often but not exclusively, an amino acid common at this position in human germline antibody sequences may be used. Human germline sequences are disclosed in Tomlinson, I. A. et al. (1992) J. Mol. Biol. 227:776-798; Cook, G. P. et al. (1995) Immunol. Today Vol. 16 (5): 237-242; Chothia, D. et al. (1992) J. Mol. Bio. 227:799-817. The V BASE directory provides a comprehensive directory of human immunoglobulin variable region sequences (compiled by Tomlinson, I. A. et al. MRC Centre for Protein Engineering, Cambridge, UK). After the deimmunized V_{H} and V_{L} sequences are constructed, the mutagenized variable sequence can, optionally, be fused to a human constant region.

In other embodiments, the antibody or antigen-binding fragment thereof can have at least one, two, and preferably three CDRs from the light or heavy chain variable region of the J591 antibody produced by the cell line having ATCC Accession Number HB-12126 or the deimmunized J591 (deJ591) antibody produced by the cell line having ATCC Accession Number PTA-3709.

In other embodiments, the antibody or antigen-binding fragment thereof can have at least one, two and preferably three CDRs from the light or heavy chain variable region of the antibody J415 produced by the cell line having ATCC Accession Number HB-12109 or the deimmunized J415 produced by a cell line having ATCC Accession Number PTA-4174.

In still other embodiments, the antibody or antigen-binding fragment thereof can have at least one, two and preferably three CDRs from the light or heavy chain variable region of the antibody J533 produced by the cell line having ATCC Accession Number HB-12127 or the antibody E99 produced by a cell line having ATCC Accession Number HB-12101.

In some embodiments, the antibody or antigen binding fragment thereof binds all or part of the epitope of an antibody described herein, e.g., J591 (produced by the hybridoma HB-12126 deposited at the ATCC), E99 (produced by the hybridoma HB-12101 deposited at the ATCC), J415 (produced by the hybridoma HB-12107 deposited at the ATCC), and J533 (produced by the hybridoma HB-12127 deposited at the ATCC). The antibody or antigen binding fragment thereof can inhibit, e.g., competitively inhibit, the binding of an antibody described herein, e.g., J591, E99, J415, and J533, to human PSMA. In some embodiments, the antibody or antigen binding fragment thereof binds to the epitope recognized by J415. In some embodiments, the antibody or antigen binding fragment thereof binds to the epitope recognized by J591. In some embodiments, the antibody or antigen binding fragment thereof binds to the epitope of E99. In some embodiments, the antibody or antigen binding fragment thereof binds to the epitope recognized by J533.

Whether two antibodies or antigen binding fragments thereof are capable of specifically binding to the same or overlapping epitopes can be determined using Scatchard analysis and/or competitive binding assays. "Specific binding" of an antibody or antigen binding fragment thereof means that the antibody exhibits sufficient affinity for antigen or a preferred epitope and, preferably, does not exhibit significant crossreactivity. "Specific binding" includes antibody binding with an affinity of at least 10⁷, 10⁸, 10⁹, or 10¹⁰ M⁻¹. An antibody or antigen binding fragment thereof that does not exhibit significant crossreactivity is one that will not appreciably bind to an undesirable entity (e.g., an undesirable proteinaceous entity) under conditions suitable to measure antibody specificity. For example, an antibody or antigen binding fragment thereof that specifically binds to PSMA will appreciably bind PSMA but will not significantly react with non-PSMA proteins or peptides. An antibody of antigen binding fragment thereof specific for a preferred epitope will, for example, not significantly cross react or with or competitively inhibit the binding to remote epitopes on the same protein or peptide. Antibodies or antigen binding fragments thereof that recognize the same epitope can be identified in a simple immunoassay showing the ability of one antibody to block the binding of another antibody to a target antigen, e.g., a competitive binding assay. Competitive binding is determined in an assay in which the antibody under test inhibits specific binding of a reference antibody to a common antigen, such as PSMA. Numerous types of competitive binding assays are known, for example: solid phase direct or indirect radioimmunoassay (RIA), solid phase direct or indirect enzyme immunoassay (EIA), sandwich competition assay (see Stahli et al., Methods in Enzymology 9:242 (1983) see also Kim, et al., Infect. Immun. 57:944 (1989)); solid phase direct biotin-avidin EIA (see Kirkland et al., J. Immunol. 137:3614 (1986)); solid phase direct labeled assay, solid phase direct labeled sandwich assay (see Harlow and Lane, Antibodies: A Laboratory Manual, Cold Spring Harbor Press (1988) pp 567-569 and 583); solid phase direct label RIA using 1-125 label (see Morel et al., Mol. Immunol, 25(1):7 (1988)); solid phase direct biotin-avidin EIA (Cheung et al., Virology 176:546 (1990)); direct labeled RIA. (Moldenhauer et al., Scand. J. Immunol. 32:77 (1990); and (Belanger L., Sylvestre C. and Dufour D. (1973)). Typically, such an assay involves the use of purified antigen bound to a solid surface or cells bearing either of these, an unlabeled test immunoglobulin and a labeled reference immunoglobulin. Competitive inhibition is measured by determining the amount of label bound to the solid surface or cells in the presence of the test antibody.

In some embodiments of the methods and kits provided herein, binding of the antibody or antigen binding fragment thereof to PSMA inhibits enzymatic activity of PSMA. PSMA, also known as NAALADase and human glutamate carboxypeptidase II ("GCP II"), possesses enzymatic activity which catalyzes the hydrolysis of the neuropeptide N-acetyl-aspartyl-glutamate ("NAAG") to N-acetyl-aspartate ("NAA") and glutamate. PSMA enzyme activity (also referred to herein as folate hydrolase activity) can be detected or measured as described below. As used herein "inhibits enzymatic activity" includes partial inhibition of folate hydrolase activity.

Also provided herein are methods of selecting or producing monoclonal antibodies that inhibit PSMA enzymatic activity. In some embodiments of selecting or producing monoclonal antibodies, the method comprises the steps of generating hybridoma cells using antibody-producing cells obtained from an animal that has been immunized with PSMA, determining whether the hybridoma cells produce antibodies that are capable of inhibiting PSMA enzymatic activity, thereby selecting a monoclonal antibody that inhibits PSMA enzymatic activity. In other embodiments of selecting or producing monoclonal antibodies hybridoma cells that produce antibodies capable of binding to extracellular PSMA are generated and antibody produced by the hybridoma cells are tested for the ability to inhibit PSMA dependent hydrolysis of a PSMA substrate in vitro. Monoclonal antibodies can be produced by a variety of techniques, including somatic cell hybridization technique of Kohler and Milstein, Nature 256: 495 (1975). See generally, Harlow, E. and Lane, D. (1988) Antibodies: A Laboratory Manual, Cold Spring Harbor Laboratory Press, Cold Spring Harbor, N.Y.

Useful immunogens for producing antibodies include PSMA (e.g., human PSMA-)-bearing cells (e.g., a prostate tumor cell line, e.g., LNCaP cells or MDA-Pca2b or LAPC4 cells, or fresh or frozen prostate tumor cells); membrane fractions of PSMA-expressing cells (e.g., a prostate tumor cell line, e.g., LNCaP cells, or fresh or frozen prostate tumor cells; PSMA-expressing vascular endothelial cells); isolated or purified PSMA, e.g., human PSMA protein (e.g., biochemically isolated PSMA, or a portion thereof, e.g., the extracellular domain of PSMA). Techniques for generating antibodies to PSMA are described in U.S. Pat. No. 6,107,090, U.S. Pat. No. 6,136,311, the contents of all of which are expressly incorporated by reference.

Antibodies that are capable of inhibiting PSMA enzymatic activity can be produced by first screening any antibodies produced directly for enzymatic activity, for example, using a PSMA enzyme substrate as described above.

In another embodiment, the antibodies are first screened for the ability to bind PSMA. Antibodies that are capable of specifically binding PSMA can be tested for PSMA enzyme activity.

Human monoclonal antibodies (mAbs) directed against human proteins can be generated using transgenic mice carrying the human immunoglobulin genes rather than the mouse immunoglobulin genes. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L. L. et al. 1994 Nature Genet. 7:13-21; Morrison, S. L. et al. 1994 Proc. Natl. Acad. Sci. USA 81:6851-6855; Bruggeman et al. 1993 Year Immunol. 7:33-40; Tuaillon et al. 1993 PNAS 90:3720-3724; Bruggeman et al. 1991 Eur J Immunol 21:1323-1326).

Antibodies or antigen binding fragments thereof useful in the present invention may also be recombinant antibodies produced by host cells transformed with DNA encoding immunoglobulin light and heavy chains of a desired antibody. Recombinant antibodies may be produced by known genetic engineering techniques. For example, recombinant antibodies may be produced by cloning a nucleotide sequence, e.g., a cDNA or genomic DNA, encoding the immunoglobulin light and heavy chains of the desired antibody. The nucleotide sequence encoding those polypeptides is then inserted into expression vectors so that both genes are operatively linked to their own transcriptional and translational expression control sequences. The expression vector and expression control sequences are chosen to be compatible with the expression host cell used. Typically, both genes are inserted into the same expression vector. Prokaryotic or eukaryotic host cells may be used.

Expression in eukaryotic host cells is preferred because such cells are more likely than prokaryotic cells to assemble and secrete a properly folded and immunologically active antibody. However, any antibody produced that is inactive due to improper folding may be renaturable according to well known methods (Kim and Baldwin, "Specific Intermediates in the Folding Reactions of Small Proteins and the Mechanism of Protein Folding", Ann. Rev. Biochem. 51, pp. 459-89 (1982)). It is possible that the host cells will produce portions of intact antibodies, such as light chain dimers or heavy chain dimers, which also are included in antibody or antigen binding portion thereof.

It will be understood that variations on the above procedure are useful for the methods and compositions provided herein. For example, it may be desired to transform a host cell with DNA encoding either the light chain or the heavy chain (but not both) of an antibody. Recombinant DNA technology may also be used to remove some or all of the DNA encoding either or both of the light and heavy chains that is not necessary for PSMA binding, e.g., the constant region may be modified by, for example, deleting specific amino acids. The molecules expressed from such truncated DNA molecules are useful for the methods and compositions described herein. In addition, bifunctional antibodies may be produced in which one heavy and one light chain are anti-PSMA antibody and the other heavy and light chain are specific for an antigen other than PSMA, or another epitope of PSMA.

Chimeric antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted.

An antibody or an immunoglobulin chain can be humanized by methods known in the art. Once the murine antibodies are obtained, the variable regions can be sequenced. The location of the CDRs and framework residues can be determined. The light and heavy chain variable regions can, optionally, be ligated to corresponding constant regions.

Humanized or CDR-grafted antibody molecules or immunoglobulins can be produced by CDR-grafting or CDR substitution, wherein one, two, or all CDRs of an immunoglobulin chain can be replaced. See e.g., U.S. Pat. No. 5,225,539; Jones et al. 1986 Nature 321:552-525; Verhoeyan et al. 1988 Science 239:1534; Beidler et al. 1988 J. Immunol. 141:4053-4060; Winter U.S. Pat. No. 5,225,539.

All of the CDRs of a particular human antibody may be replaced with at least a portion of a non-human CDR from an antibody or antigen binding fragment thereof that is capable of binding the antigen of interest, or only some of the CDRs may be replaced with non-human CDRs. It is only necessary to replace the number of CDRs required for binding of the humanized antibody to the antigen of interest.

Humanized antibodies can be generated by replacing sequences of the Fv variable region that are not directly involved in antigen binding with equivalent sequences from human Fv variable regions. General methods for generating humanized antibodies are provided by Morrison, S. L., 1985, Science 229:1202-1207, by Oi et al., 1986, BioTechniques 4:214, and by Queen et al. U.S. Pat. No. 5,585,089, U.S. Pat. No. 5,693,761 and U.S. Pat. No. 5,693,762. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against the antigen of interest. The recombinant DNA encoding the humanized antibody, or fragment thereof, can then be cloned into an appropriate expression vector.

The anti-PSMA antibody, or antigen fragment thereof, may also be modified by specific deletion of human T cell epitopes or "deimmunization" by the methods disclosed in WO 98/52976 and WO 00/34317. Briefly, the murine heavy and light chain variable regions of an anti-PSMA antibody can be analyzed for peptides that bind to MHC Class II; these peptides represent potential T-cell epitopes.

### EXAMPLES

### Effect of Hormone Withdrawal on PSMA Expression.

Prostate cancer cell lines, LNCaP and A-Pca-2b were grown in standard medium containing 10% fetal calf serum (FCS) or 10% FCS that had been charcoal stripped (CS-FCS). FCS contains steroid hormones including androgens, whereas CS-FCS does not have steroid hormones including androgens, because charcoal-stripping removes some components of serum including steroid hormones. Charcoal-stripping is the standard method for removing hormones from media. The various curves represent PSMA levels after growing cells for the specified period of time in the CS-FCS media. The PSMA levels are detected by contacting the cells with J591, an anti-PSMA antibody, followed by a labeled secondary antibody that recognizes J591. The labeled cells are then analyzed by FACS.

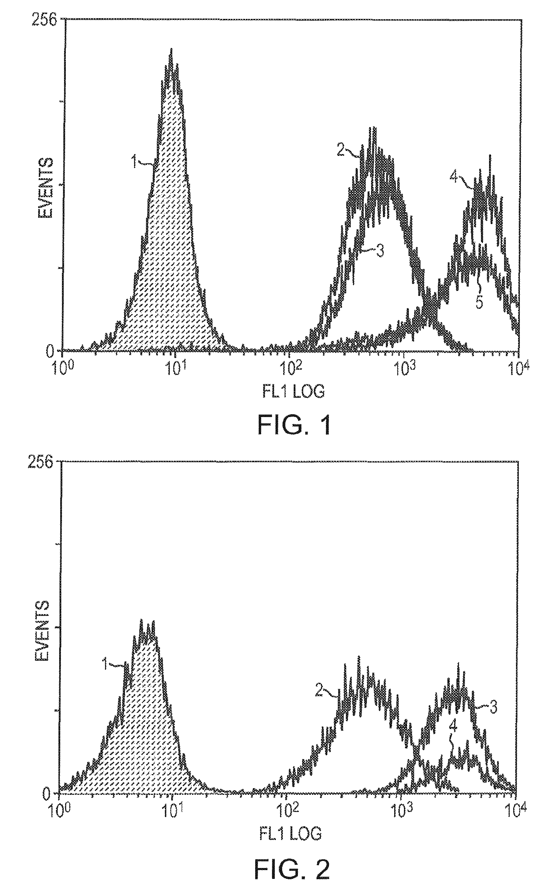

FIG. 1 shows that at 1 week (line 3), there is very little increase in PSMA expression in LNCaP cells. However, by 2 weeks (line 4) there is what equates to a 9-fold increase in PSMA expression. There is little upregulation in PSMA expression at 3 weeks over 2 weeks (line 5) as demonstrated that the peak has not moved further to the right. The 3 week peak is lower on the y-axis because some of the cells are dying due to loss of hormonal supplementation and are not measured in this assay.

FIG. 2 shows that MDA-Pca-2b has a very similar PSMA expression pattern in the absence of hormone withdrawal. Line 1 represents the negative control, line 2 represents the expression level in standard media with 10% FCS, line 3 represents PSMA expression after culturing the cells for 2 weeks in CS-FCS media, and line 4 represents PSMA expression after culturing the cells for 3 weeks in CS-FCS media.

### Effect of Folic Acid Concentration on Cell Growth Rate.

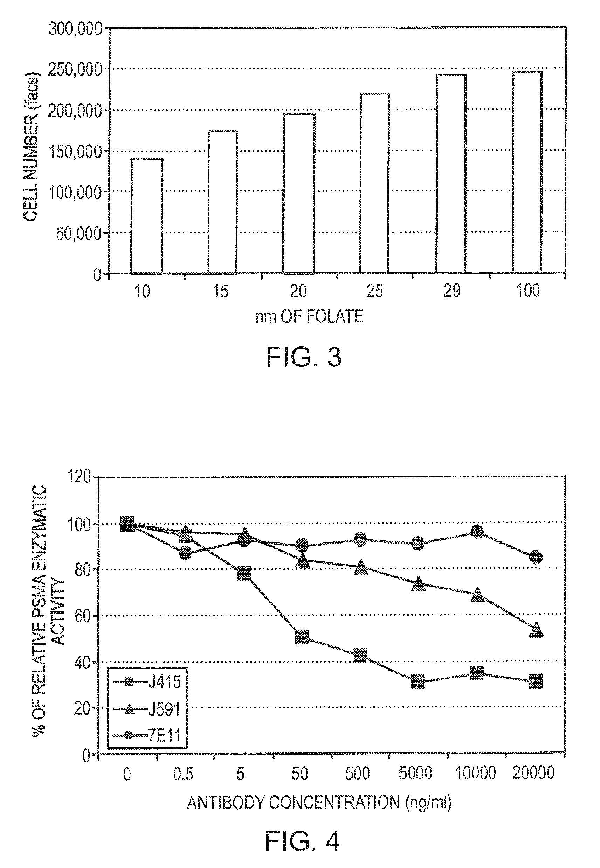

As demonstrated in FIG. 3, the growth rate of prostate cancer (PC) cells increases in direct proportion to available folate, indicating that, if PC cells can acquire greater amounts of folate, they can grow faster. Increasing the folate level above physiological can increase the growth rate by almost 2-fold. In cell lines that express PSMA (such as LNCaP), the same growth increase is also found if folate is provided in the form of 'natural' or dietary folate (e.g., polyglutamated folate) which is digested to monoglutamated folate by PSMA. The latter (monoglutamated folate) can cross the cell membrane whereas polyglutamated folate cannot.

### Anti-PSMA Antibody, J415, Inhibits PSMA tic Activity.

LNCaP cells grown in standard media were incubated with the indicated concentrations of the indicated antibody. Cells were tested for PSMA enzymatic activity. Briefly, PSMA was incubated with a radiolabeled substrate such as NAAG. After the incubation, the reaction mixture was run over a column that retains the labeled glutamate product but not the substrate. The amount of labeled glutamate was measured in a gamma counter. The amount of labeled glutamate measured is proportional to the amount of enzymatic activity. The PSMA can be purified, recombinant or cell-associated. One can also assay for inhibition by including inhibitor in the first incubation step and by using appropriate positive and negative controls to calculate the level of inhibition. As demonstrated by FIG. 4, antibody J415 maximally inhibits PSMA/FolH1 enzymatic activity at a concentration of 5,000 ng/ml (5 ug/ml). Maximal inhibition is approximately 70%. The J591 also inhibits PSMA/FolH1 enzymatic activity but less so than J415. The 7E11 antibody, which binds an intracellular epitope of PSMA, distant from the enzymatic site, has no PSMA/FolH1 inhibitory activity.

As demonstrated by FIG. 4, antibody J415 maximally inhibits PSMA/FolH1 enzymatic activity at a concentration of 5,000 ng/ml (5 ug/ml). Maximal inhibition is approximately 70%. The J591 also inhibits PSMA/FolH1 enzymatic activity. The 7E11 antibody, which binds an intracellular epitope of PSMA, distant from the enzymatic site, has no PSMA/FolH1 inhibitory activity. Thus, inhibiting the PSMA (folate hydrolase) enzymatic activity, most efficiently accomplished by J415, diminishes the ability of PSMA expressing cells to convert cell impermeant folylpolyglutamate into cell permeant folylmonoglutamate thereby lowering the intracellular availability of folate for cell metabolism and growth. PSMA Expression is Upregulated in Response to Lowered Folate in the Media.

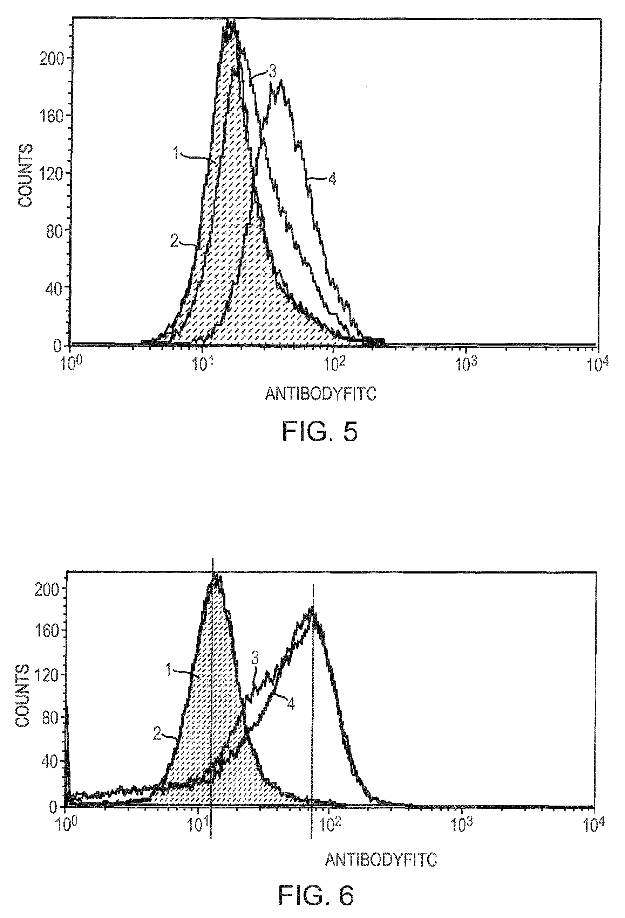

According to the World Health Organization, physiological level of serum folate is 12 nmol/L. Standard tissue culture media contains approx 150x the concentration of folate found in human serum/plasma. As demonstrated herein, changing the concentration of folate in the media to physiological levels upregulates the level of PSMA expression in various kidney and prostate cancer cell lines. FIGs. 5-11 show the PSMA expression levels of different cell lines were grown for three weeks in standard media (containing 150x physiological concentration of folate; lines 2), standard media with only 50 folate (lines 3), or standard media with only 10 nM folate (lines 4). The cells were labeled with anti-PSMA antibody J591 followed by a fluorescently labeled secondary antibody that recognizes J591. Lines 1 show the cells labeled with a negative isotype control antibody followed by the labeled secondary antibody.

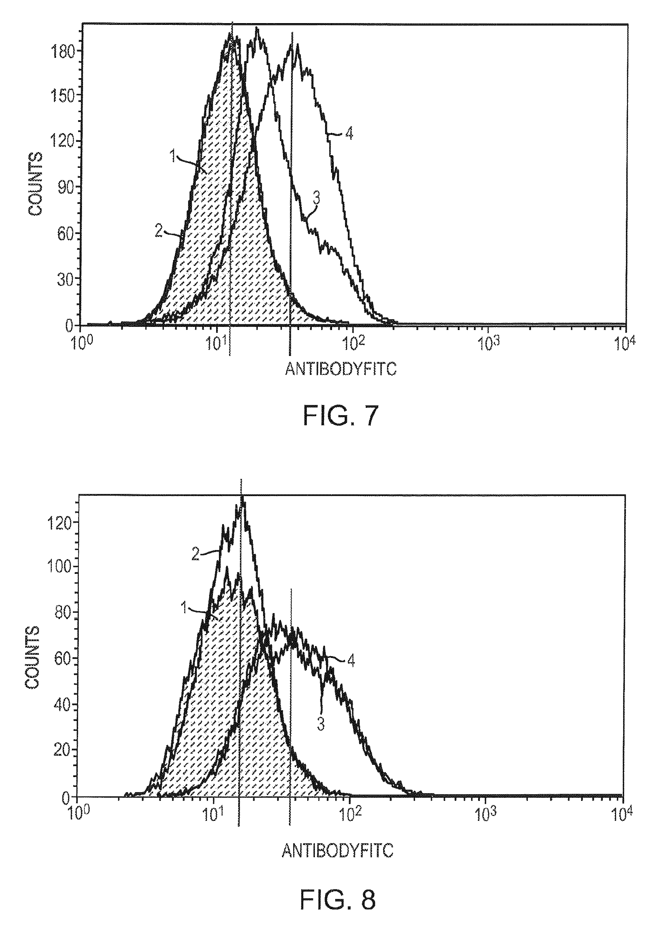

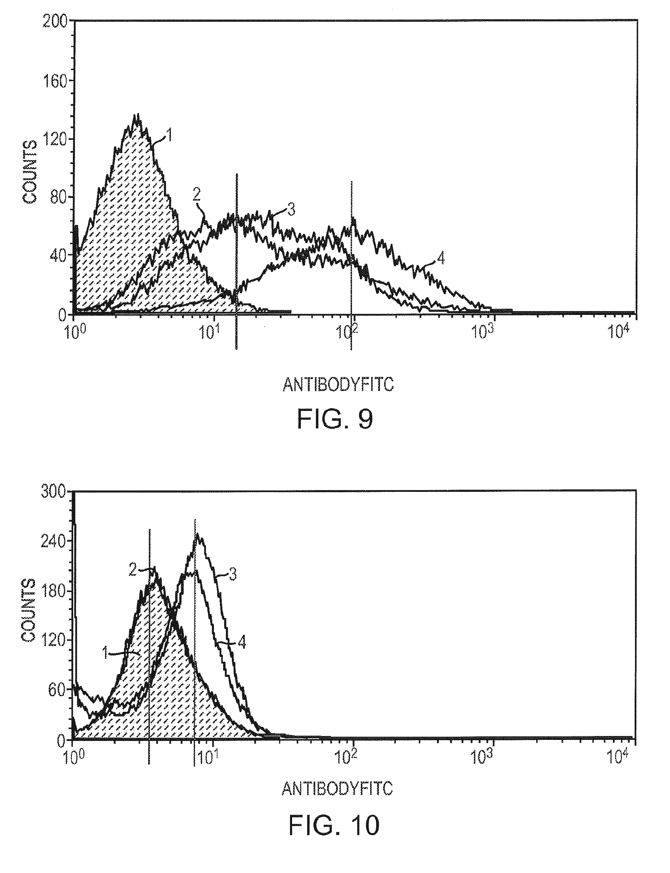

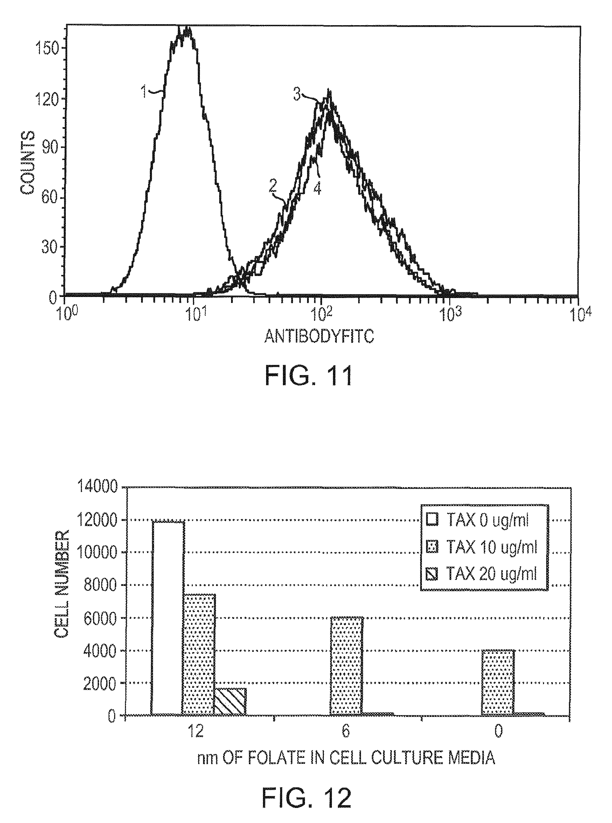

FIG. 5 shows that lowering the folate levels to 10 nM resulted in a 250% increase in PSMA expression by the human kidney cancer cell line, SK-RC-31, FIG. 6 shows that lowering the folate levels to 10-50 nM resulted in a 650% increase in PSMA expression by the human kidney cancer cell line, SK-RC-42. FIG. 7 shows that lowering the folate levels to 10 nM resulted in a 250% increase in PSMA expression by the human kidney cancer cell line, SK-RC-39. FIG. 8 shows that lowering the folate levels to 50 nM or less resulted in a 225% increase in PSMA expression by the human kidney cancer cell line, SK-RC-06. FIG. 9 shows that lowering the folate levels to 10 nM resulted in a 950% increase in PSMA expression by the human prostate cancer cell line, Cwr22rv1. FIG. 10 shows that lowering the folate levels to 50 nM or less resulted in a 400% increase in PSMA expression by the human prostate cancer cell line, PC3. FIG. 11 shows that lowering folate levels in the media did not cause upregulation of PSMA expression by the human prostate cancer cell line, LNCaP, a cell line that constitutively expresses very high levels of PSMA.

Line 4 'low folate' approximates the normal physiological folate level found in humans. Three weeks was chosen to allow previous stores of folate to be depleted and was based-on the timing of the increase in PSMA expression.

### Decreased Folate Levels Potentiates Docetaxel Inhibition of LNCaP Cell Growth.

Docetaxel (taxotere) at concentrations of 10 or 20 ug/ml was incubated with LNCaP cells for 72 hours and at different concentrations of folate supplied in the form of folic acid from culture medium. Cells were counted after 1 week. As shown in FIG. 12, decreasing folate levels increases the cell killing/inhibition of growth resulting from docetaxel, an approved chemotherapeutic agent used in prostate cancer and other cancers. The open bar is the control without treatment with docetaxel, the speckled bars are cells treated with 10 ug/ml docetaxel together with the indicated concentration of folate, and the striped bars are cells treated with 20 ug/ml docetaxel together with the indicated concentration of folate. The indicated concentrations of folate are over and above the physiological level of folate provided by the folate present in fetal calf serum (for example, 12 nm folate represents 12 nm folate above the physiological concentration). As demonstrated by FIG. 12, lowering folate levels increases the effectiveness of taxotere. It is likely that decreased folate availability to cancer cells would enhance the anti-tumor effect of other cytotoxic, cytostatic and hormonal.

### Treatment of a Murine Prostate Cancer Tumor Model with an Antibody that Inhibits PSMA Activity Slows Tumor Growth by 50%.

Nude mice, eight per group, were injected with human LNCaP prostate cancer cells in matrigel on day 0. Mouse serum folate levels are on average 10-40 x that of humans. Therefore, all mice were maintained on a diet with no added folate and with an antibiotic to prevent intestinal bacteria from synthesizing folate in order to lower serum concentrations of folate in the mice. Two groups of mice (A&B) were fed folylpolyglutamate in their drinking water. Folylpolyglutamate is the form of folate found naturally in food, and as described previously, does not cross cell the membrane. PSMA removes the glutamates from folylpolyglutamate to produce folylmonoglutamate, which can cross the cell membrane. The murine intake and synthesis of folate was controlled to mimic human folate physiology as closely as possible.

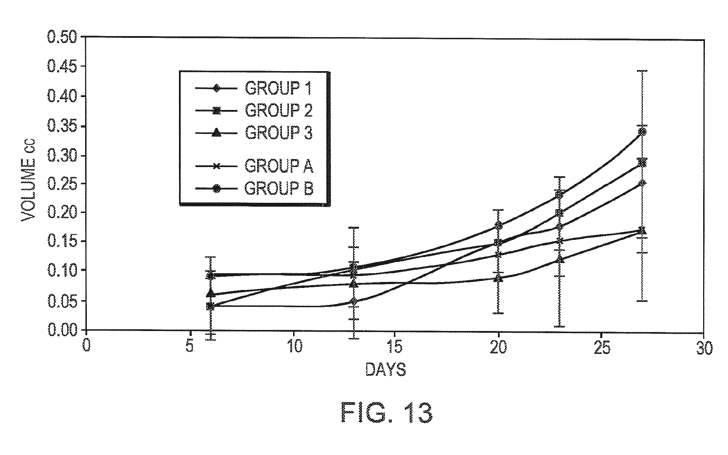

Animals were treated with 3 different anti-PSMA antibodies, J591, 7E11 and J415, on day 1 and every 2 weeks thereafter. Group 1 received J591, Group 2 received 7E11, and Group 3 received J415. The antibodies were unlabeled, naked antibodies. Doses were 250 ug per dose per mouse. Tumor measurements were done with calipers by laboratory assistants blinded to the treatment received by the respective groups. Animals in Group B are the control group with no active treatment. As shown in FIG. 13, 7E11, which has no, and J591, which has minimal ability to inhibit PSMA/FOLH1 enzymatic activity, have little impact on tumor growth. However, two groups of J415-treated mice, 1 receiving folylpolyglutamate (Group A) and 1 without (Group 3), both show tumor growth reduced by 50%.

Examples of methods of labeling antibodies or antigen binding fragments thereof with radiolabels or cytotoxic agents are found in US Published applications 20060088539 and 20060275212 to Bander, in particular in the Examples, the teachings of these applications are incoporated herein in their entirety.

The technology provided herein may be embodied in other specific forms without departing from the spirit or essential characteristics thereof. The foregoing embodiments are therefore to be considered in all respects illustrative rather than limiting on the technology described herein. Scope of the technology provided herein is thus indicated by the appended claims rather than by the foregoing description, and all changes which come within the meaning and range of equivalency of the claims are therefore intended to be embraced therein.

### The invention is further defined in the following clauses:

1. Use of a hormone that is suitable for hormone therapy and an antibody or antigen binding fragment thereof that is capable of binding to an extracellular domain of PSMA wherein the antibody or antigen binding fragment thereof is conjugated to Lutetium-177 in the manufacture of a medicament for the treatment of early stage prostate cancer in a patient,
   wherein a first dose of the antibody or antigen binding fragment thereof is to be administered one day to four weeks after hormonal therapy has begun or
   wherein a first dose of the antibody or antigen binding fragment thereof is to be administered to the patient one day to four weeks after serum testosterone levels of the patient have reached 50 ng/mL or less.
2. The use of any one of clauses 1 and 3-7, wherein the patient has elevated prostate specific antigen (PSA) levels and lacks soft tissue disease greater than 0.9 cm in diameter.
3. The use of any one of clauses 1, 2, and 4-7, wherein the antibody or antigen binding fragment thereof is conjugated to an agent or is capable of eliciting an antibody-dependent cellular cytotoxic response.
4. The use of any one of clauses 1-3 and 5-7, wherein the antibody or antigen binding fragment thereof is capable of binding to an extracellular domain of PSMA and inhibiting enzymatic activity of the PSMA.
5. The use of any one of clauses 1-4, 6, and 7, further comprising measuring surface levels of PSMA on PSMA expressing cells of the patient and wherein the first dose of the antibody or antigen binding fragment thereof is administered one to five days after cell surface levels of PSMA on PSMA expressing cells of the patient has increased by five fold or more.
6. The use of any one of clauses 1-5 and 7, further comprising measuring surface levels of PSMA on PSMA expressing cells of the patient and wherein the first dose of the antibody or antigen binding fragment thereof is administered within one day to five days after cell surface levels of PSMA on PSMA expressing cells has increased by nine fold or more.
7. The use of any one of clauses 1-6, further comprising monitoring serum testosterone levels in the patient.
8. Use of an unlabeled antibody or antigen binding fragment thereof that is capable of binding to an extracellular domain of PSMA and inhibiting enzymatic activity of the PSMA in the manufacture of a medicament for the treatment of cancer in a patient, wherein the medicament is to be used in conjunction with restricting intake of folate by the patient.
9. The use of any one of clauses 8 and 10-27, wherein folate intake by the patient is restricted by prescribing dietary supplements that contain folate.
10. The use of any one of clauses 8, 9, and 11-27, wherein folate intake by the patient is restricted such that folate intake is 400 µg per day or less, or such that serum level of folate in the patient is 10 nmol/L or less, or such that red blood cell (RBC) folate level in the patient is 300 nmol/L or less.
11. The use of any one of clauses 8-10 and 12-27, wherein the cancer is prostate cancer.
12. The use of any one of clauses 8-11 and 13-27, wherein intake of folate by the patient is limited prior to administering a first dose of the unlabeled antibody or antigen binding fragment thereof.
13. The use of any one of clauses 8-12 and 14-27, wherein a first dose of the unlabeled antibody or antigen binding fragment thereof is to be administered within 24 hours of restricting intake of folate by the patient.
14. The use of any one of clauses 8-13 and 15-27, wherein a first dose of the unlabeled antibody or antigen binding fragment thereof is to be administered two weeks after restricting folate intake by the patient begins.
15. The use of any one of clauses 8-14 and 16-27, wherein the unlabeled antibody or antigen binding fragment thereof is administered in sufficient amount to maintain a serum concentration of 5 µg/mL or higher of antibody or antigen binding fragment thereof in the patient's serum or plasma.
16. The use of any one of clauses 8-15 and 17-27, wherein the unlabeled antibody or antigen binding fragment thereof is an antibody and wherein the antibody is administered in sufficient amount to achieve a serum concentration of about 50 µg/mL of antibody in the patient.
17. The use of any one of clauses 8-16 and 18-27, wherein the unlabeled antibody or antigen binding fragment thereof is a monoclonal antibody or antigen binding fragment thereof produced by a hybridoma deposited under ATCC deposit accession number HB-12109.
18. The use of any one of clauses 8-17 and 19-27, further comprising measuring blood levels of the unlabeled antibody or antigen binding fragment thereof in the patient. and adjusting the amount of antibody or antigen binding fragment thereof administered to maintain a serum concentration of 5 µg/mL or higher.
19. The use of any one of clauses 8-18 and 20-27, further comprising measuring blood levels of folate in the patient.
20. The use of clause 19, further comprising adjusting the intake of folate by the patient based on the blood levels of folate such that serum level of folate in the patient is 10 nmol/L or less or such that red blood cell (RBC) folate level in the patient is 300 nmol/L or less.
21. The use of any one of clauses 8-20 and 22-27, further comprising administering a chemotherapeutic agent to the patient.
22. The use of clause 21, wherein the chemotherapeutic agent is an antagonist of folate metabolism.
23. The use of clause 22, wherein the antagonist is administered at a dose that reduces folate metabolic activity by no more than about 40%.
24. The use of clause 21, wherein the chemotherapeutic agent is docetaxel.
25. The use of any one of clauses 8-24, 26, and 27, further comprising measuring PSMA activity of PSMA expressing cells of a patient.
26. The use of any one of clauses 8-25 and 27, further comprising administering hormonal therapy to the patient.
27. The use of clause 26, wherein hormonal therapy is begun within 24 hours of administering a first dose of the unlabeled antibody or antigen binding fragment thereof to the patient.
28. A kit for treating cancer comprising:
   a) an antibody or antigen binding fragment thereof that is capable of binding to an extracellular domain of PSMA and inhibiting enzymatic activity of the PSMA, and
   b) instructions for restricting intake of folate by the patent.
29. The kit of clause 28, wherein the antibody or antigen binding fragment thereof is conjugated to a cytotoxic agent or is capable of eliciting an antibody-dependent cellular cytotoxic response.
30. A kit for monitoring cancer therapy in a patient comprising:
   a) a tissue or blood collection apparatus;
   b) a PSMA activity detection reagent, and
   c) instructions for testing tissue or blood sample obtained from the patient using the PSMA activity detection reagent.
31. A kit for monitoring cancer therapy in a patient comprising:
   a) a blood collection apparatus;
   b) a folate detection reagent, and
   c) instructions for testing red blood cells or serum obtained from the patient using the folate detection reagent.
32. The kit of clause 31, further comprising instructions for reducing folate intake by the patient based on folate levels detected using the kit.
33. A method of treating prostate cancer in a patient comprising the steps of:
   administering hormonal therapy to the patient, wherein the patient has been diagnosed with early stage prostate cancer; and
   administering an antibody or antigen binding fragment thereof that is capable of binding to an extracellular domain of PSMA wherein the antibody or antigen binding fragment thereof is conjugated to Lutetium-177,
   wherein a first dose of the antibody or antigen binding fragment thereof is administered one day to four weeks after hormonal therapy is begun.
34. The method of clause 33, wherein the patient has elevated prostate specific antigen (PSA) levels and lacks soft tissue disease greater than 0.9 cm in diameter.
35. A method of treating prostate cancer in a patient comprising the steps of:
   administering hormonal therapy to the patient, and
   administering an antibody or antigen binding fragment thereof to a patient, wherein the antibody or antigen binding fragment thereof is capable of binding to an extracellular domain of PSMA, and wherein a first dose of the antibody or antigen binding fragment thereof is administered to the patient one day to four weeks after serum testosterone levels of the patient have reached 50 ng/mL or less.
36. The method of any one of clauses 35 and 37-42, wherein the antibody or antigen binding fragment thereof is conjugated to an agent or is capable of eliciting an antibody-dependent cellular cytotoxic response.
37. The method of any one of clauses 35, 36 and 38-42, wherein the antibody or antigen binding fragment thereof is capable of binding to an extracellular domain of PSMA and inhibiting enzymatic activity of the PSMA.
38. The method of any one of clauses 35-37 and 39-42, further comprising measuring surface levels of PSMA on PSMA expressing cells of the patient.
39. The method of any one of clauses 35-38 and 40-42, wherein the first dose of the antibody or antigen binding fragment thereof is administered when cell surface levels of PSMA on PSMA expressing cells of the patient has increased by five fold or more.
40. The method of any one of clauses 35-39, 41, and 42, further comprising measuring surface levels of PSMA on PSMA expressing cells of the patient and wherein the first dose of the antibody or antigen binding fragment thereof is administered one to five days after cell surface levels of PSMA on PSMA expressing cells of the patient has increased by five fold or more.
41. The method of any one of clauses 35-40 and 42, further comprising measuring surface levels of PSMA on PSMA expressing cells of the patient and wherein the first dose of the antibody or antigen binding fragment thereof is administered within one day to five days after cell surface levels of PSMA on PSMA expressing cells has increased by nine fold or more.
42. The method of any one of clauses 35-41, further comprising monitoring serum testosterone levels in the patient.
43. A method of treating cancer in a patient with an unlabeled antibody or antigen binding fragment thereof comprising the steps of:
   administering an unlabeled antibody or antigen binding fragment thereof to a patient, wherein the unlabeled antibody or antigen binding fragment thereof is capable of binding to an extracellular domain of PSMA and inhibiting enzymatic activity of the PSMA, and
   restricting intake of folate by the patient.
44. The method of any one of clauses 43 and 45-62, wherein folate intake by the patient is restricted by proscribing dietary supplements that contain folate.
45. The method of any one of clauses 43, 44, and 46-62, wherein folate intake by the patient is restricted such that folate intake is 400 µg per day or less, or such that serum level of folate in the patient is 10 nmol/L or less, or such that red blood cell (RBC) folate level in the patient is 300 nmol/L or less.
46. The method of any one of clauses 43-45 and 47-62, wherein the cancer is prostate cancer.
47. The method of any one of clauses 43-46 and 48-62, wherein intake of folate by the patient is limited prior to administering a first dose of the unlabeled antibody or antigen binding fragment thereof.
48. The method of any one of clauses 43-47 and 49-62, wherein a first dose of the unlabeled antibody or antigen binding fragment thereof is administered within 24 hours of restricting intake of folate by the patient.
49. The method of any one of clauses 43-48 and 50-62, wherein a first dose of the unlabeled antibody or antigen binding fragment thereof is administered two weeks after restricting folate intake by the patient begins.
50. The method of any one of clauses 43-49 and 51-62, wherein the unlabeled antibody or antigen binding fragment thereof is administered in sufficient amount to maintain a serum concentration of 5 µg/mL or higher of antibody or antigen binding fragment thereof in the patient's serum or plasma.
51. The method of any one of clauses 43-50 and 52-62, wherein the unlabeled antibody or antigen binding fragment thereof is an antibody and wherein the antibody is administered in sufficient amount to achieve a serum concentration of about 50 µg/mL of antibody in the patient.
52. The method of any one of clauses 43-51 and 53-62, wherein the unlabeled antibody or antigen binding fragment thereof is a monoclonal antibody or antigen binding fragment thereof produced by a hybridoma deposited under ATCC deposit accession number HB-12109.
53. The method of any one of clauses 43-52 and 54-62, further comprising measuring blood levels of the unlabeled antibody or antigen binding fragment thereof in the patient. and adjusting the amount of antibody or antigen binding fragment thereof administered to maintain a serum concentration of 5 µg/mL or higher.
54. The method of any one of clauses 43-53 and 55-62, further comprising measuring blood levels of folate in the patient.
55. The method of clause 54, further comprising adjusting the intake of folate by the patient based on the blood levels of folate such that serum level of folate in the patient is 10 nmol/L or less or such that red blood cell (RBC) folate level in the patient is 300 nmol/L or less.
56. The method of any one of clauses 43-55 and 57-62, further comprising administering a chemotherapeutic agent to the patient.
57. The method of clause 56, wherein the chemotherapeutic agent is an antagonist of folate metabolism.
58. The method of any one of clauses 43-57 and 59-62, wherein the antagonist is administered at a dose that reduces folate metabolic activity by no more than about 40%.
59. The method of clause 56, wherein the chemotherapeutic agent is docetaxel.
60. The method of any one of clauses 43-59 and 61-62, further comprising measuring PSMA activity of PSMA expressing cells of a patient.
61. The method of any one of clauses 43-60 and 62-62, further comprising administering hormonal therapy to the patient.
62. The method of clause 61, wherein hormonal therapy is begun within 24 hours of administering a first dose of the unlabeled antibody or antigen binding fragment thereof to the patient.
63. A method of monitoring cancer therapy in a patient comprising measuring blood levels of folate in the patient, wherein folate intake by the patient is being restricted to 400 µg per day or less, or such that serum level of folate in the patient is 10 nmol/L or less, or such that red blood cell (RBC) folate level in the patient is 300 nmol/L or less, and wherein the patient has received at least one dose of an antibody or antigen binding fragment thereof that is capable of binding to an extracellular domain of PSMA and inhibiting enzymatic activity of the PSMA.
64. A method of monitoring cancer therapy in a patient comprising measuring PSMA activity of PSMA expressing cells of a patient, wherein folate intake by the patient is being restricted to 400 µg per day or less, or such that serum level of folate in the patient is 10 nmol/L or less, or such that red blood cell (RBC) folate level in the patient is 300 nmol/L or less, and wherein the patient has received at least one dose of an antibody or antigen binding fragment thereof that is capable of binding to an extracellular domain of PSMA and inhibiting enzymatic activity of the PSMA.
65. A kit for treating early stage prostate cancer in a patient comprising:
   an antibody or antigen binding fragment thereof that is capable of binding to an extracellular domain of PSMA wherein the antibody or antigen binding fragment thereof is conjugated to Lutetium-177; and
   instructions for administering a first dose of the antibody or antigen binding fragment thereof either one day to four weeks after a hormonal therapy has begun or one day to four weeks after serum testosterone levels of the patient have reached 50 ng/mL or less.
66. A kit for treating cancer in a patient comprising:
   an unlabeled antibody or antigen binding fragment thereof that is capable of binding to an extracellular domain of PSMA and inhibiting enzymatic activity of the PSMA; and
   instructions for administering the unlabeled antibody or antigen binding fragment thereof in conjunction with restricting intake of folate by the patient.

## Claims

1. Use of a hormone that is suitable for hormone therapy of prostate cancer and an antibody or antigen binding fragment thereof that is capable of binding to an extracellular domain of PSMA in the manufacture of a medicament for the treatment of prostate cancer in a patient wherein a first dose of the antibody or antigen binding fragment thereof is administered to the patient one day to four weeks after hormonal therapy is begun

2. The use of claim 1, wherein the antibody or antigen binding fragment thereof is an agent capable of eliciting an antibody-dependent cellular cytotoxic response.

3. The use of any one of claims 1 to 2, wherein a first dose of the antibody or antigen binding fragment thereof is administered to the patient one day to four weeks after serum testosterone levels of the patient have reached 50 ng/mL or less.

4. The use of any one of claims 1 to 3, further comprising measuring surface levels of PSMA on PSMA expressing cells of the patient.

5. The use of any one of claims 1 to 4, wherein the first dose of the antibody or antigen binding fragment thereof is administered when cell surface levels of PSMA on PSMA expressing cells of the patient has increased by five fold or more from the baseline, pre-hormonal initiation level.

6. The use of any one of claims 1 to 5, further comprising measuring surface levels of PSMA on PSMA expressing cells of the patient and wherein the first dose of the antibody or antigen binding fragment thereof is administered one to five days after cell surface levels of PSMA on PSMA expressing cells of the patient has increased by five fold or more from the baseline, pre-hormonal initiation level.

7. The use of any one of claims 1 to 6, further comprising measuring surface levels of PSMA on PSMA expressing cells of the patient and wherein the first dose of the antibody or antigen binding fragment thereof is administered within one day to five days after cell surface levels of PSMA on PSMA expressing cells has increased by nine fold or more from the baseline, pre-hormonal initiation level.

8. The use of any one of claims 1 to 7, further comprising monitoring serum testosterone levels in the patient.
